# EUROPEAN PATENT SPECIFICATION

(11) **EP 3 672 691 B1**
(45) Date of publication and mention of the grant of the patent: **28.06.2023**
(21) Application number: 17758490.1
(22) Date of filing: 23.08.2017
(51) Int. Cl.: A61Q 5/12, A61Q 5/00, A61K 8/97

(54) **USES OF EXTRACTS OF ISOCHRYSIS SP**
VERWENDUNGEN VON ISCHRYSIS SP EXTRAKTEN
UTILISATIONS D'EXTRAITS D'ISOCHRYSIS SP

(43) Date of publication of application: 01.07.2020
(73) Proprietor: Symrise AG, 37603 Holzminden (DE)
(72) Inventor: NAKANO, Adelino, 13340-350 Indaiatuba (BR); LOURENÇO, Carolina, 06715-400 Sao Paulo (BR); PAULA, Marcia, 05020-000 Sao Paulo (BR)
(74) Representative: Eisenführ Speiser
(86) International application number: PCT/EP2017/071221
(87) International publication number: WO 2019/037843

(56) References cited:
- EP-A2- 2 168 570
- EP-B1- 2 168 570
- WO-A2-2015/191449
- DATABASE GNPD [Online] MINTEL; 30 April 2017 (2017-04-30), Oriflame: "SCALP TONIC", XP002774988, Database accession no. 4717497
- DATABASE GNPD [Online] MINTEL; 28 February 2017 (2017-02-28), Newhall Laboratories: "Revitalizing Shampoo", XP002774989, Database accession no. 4627077

## Description

The present invention primarily relates to the use of a composition consisting of or comprising an extract of Isochrysis sp., preferably of Tahitian Isochrysis, for influencing or modifying the hair fiber of human hair, wherein the use is further specified as set forth in appended claim 1. The invention furthermore relates to a method for influencing or modifying the hair fiber of human hair as specified in appended claim 11.

Further aspects of the present invention will arise from the description below, in particular from the examples, as well as from the attached patent claims.

The human hair fiber presents four main structures in its morphology. The cuticle layer is the outermost superficial layer in which up to ten layers of cuticle cells are found. Its function is to protect the inner structures of the hair and to control the penetration of molecules. The cortex is the internal portion, in which 95% of the protein content is present. It is formed by longitudinal filaments oriented in parallel to the hair shaft. The medulla is the central structure and may be present or not in the hair length. Its function in the hair fiber is still not clear but recent investigations show influence on light diffusion. Finally, there is the cell membrane complex (CMC), which is also called hair cement. It is a lipidic matrix that keeps cuticle and cortex structures together. There are three types of CMCs. The first type of CMC is between just cuticles, the second type of CMC is between the innermost cuticle layer and the cortical cells and the third CMC is between the cortical cells themselves.

The hair fiber is a dead tissue and its exposition to different cosmetic treatments and/or environmental stress leads to the loss of amino acids, lipids and proteins and consequently, to the change of the structure of the hair fiber. As a result, the hair may appear dull, is hard to comb and difficult to manage and may display a non-homogeneous color.

Usual strategies to treat the hair involve, for instance, restoring the lost material such as replenishing the hair fiber with amino acids, certain lipids and even keratin-derived molecules, treating the damage with film forming materials, lubricants and/or silicones to improve its appearance, or preventing the damage in the first place by using UV filters, antioxidants and networking materials, which are able to reduce the friction of the hair fiber with external physical treatments (like e.g. combing or brushing).

WO 2015/191449 A2 describes compositions containing microalgae or microalgal components and methods for their use as personal care products such as skin and hair care products.

Thus, the object of the present invention was to identify compounds or mixtures that are capable of influencing or modifying the hair fiber of human hair, in particular which show improved performance in terms of improving combability, shine, volume, frizz control and resistance to break of the hair fibers.

According to a first aspect of the present invention, the stated object is surprisingly achieved by the use of a composition consisting of or comprising an extract of Isochrysis sp., preferably Tahitian Isochrysis, for influencing or modifying the hair fiber of human hair, wherein the use is further specified as set forth in appended claim 1.

The patent EP 2 168 570 B1 relates, *inter alia,* to the use of extracts of cell material of Isochrysis sp., preferably of Tahitian Isochrysis, to influence or modify the growth of human hair and/or pigmentation of human hair and/or skin. In terms of the application to human hair, it describes the influence of the extracts on the growth of human hair follicles and on the metabolism of said follicles. It was demonstrated that depending on the solvent used for extraction of the cell material, the extracts either increased or reduced the growth of the tested human hair follicles. Thus, the extracts were shown to be useful - dependent on the desired result - either for stimulating or inhibiting the growth of human hair.

Said document, however, is entirely silent on the influence of the extracts of cell material of Isochrysis sp. on the fiber of human hair. It was thus surprising that during the studies underlying the present invention it was found that said extracts not only modify the growth of human hair, but also have very favorable effects on the fiber of human hair (as will be described in further detail in the following).

According to the present invention, the composition is used to
(i) improve the combability of human hair and/or
(ii) control the volume of human hair fibers and/or
(iii) avoid or reduce the frizz of human hair and/or
(iv) improve the tensile strength of human hair fibers and/or
(v) decrease the fiber breakage of human hair, preferably during or after exposure of the hair fiber to physical and/or chemical and/or environmental stress.

Hair combability relates to the force in Joules required for a comb to pass through a sample human hair tress (cf. combability test below for further details). Improved hair combability is particularly advantageous since it not only reduces the pain and potential hair loss experienced by the user during the combing process, it also minimizes the exposure of the hair fiber to further physical stress or damage.

Increased volume is the gain of body in a hair tress due to its humidity exposure or mechanical actions such as combing or friction of hair fibers. Frizz is the bristling of some fibers of hair that get apart from the tress body (fly-away effect). Many users of hair care products expect a certain increase in hair volume after using a hair care product, but do not desire excessive increase of the volume of their hair after application of said product. Moreover, they most users like to avoid frizzy hair. Thus, the use of a composition according to the invention, which controls the frizz and volume of human hair fibers is highly desirable.

Linear mechanical properties of the hair fiber include, for example, the elastic module (relating to the stiffness of the hair fiber) and the stress measured at 15% strain. It has been found that the compositions according to the present invention are able to increase the tensile strength of human hair fibers, i.e. increase the elastic module of the hair fibers and increase the stress measured at 15% strain (compositions and test methods are described further below in more detail). This is particularly advantageous, since strengthening the hair fibers is highly desirable for users of hair care products.

Hair fiber breakage of human hair may occur after exposure of the hair to certain stress factors such as physical and/or chemical and/or environmental stress. It has been found that advantageously the use of a composition according to the invention leads to a reduction of hair fiber breakage. Thus, the hair fibers become more stable and the user is able to grow longer hair before the hair fiber breaks off.

Another preferred embodiment according to the invention is a use as described herein, alternative (v), wherein the physical stress originates or originated from combing, drying, heating, curling or straightening the hair fibers, or the use of retaining means such as hair clips, hair curlers or hair ties and/or the chemical stress originates or originated from hair care products such as shampoos, conditioners, hair treatment cures, hair tonics, hair lotions, hair rinses, styling creams, hair setting compositions, styling aids, blonding compositions or hair colouring compositions and/or the environmental stress originates or originated from exposure to sun, industrial pollution, sea water or chlorinated water.

The exposure of hair fibers to the above physical stress factors may lead e.g. to the loss of cuticle cells, loss of CMC, fatty acids (such as stearic, palmitic or oleic acid), cholesterol and ceramides. It can also lead to cuticle breakage and cracks, less brightness of the hair fiber and difficulties with combing the hair.

The exposure of hair fibers to the above chemical stress factors may lead e.g. to the loss of amino acids (Tyr, Cys, Met), the loss of 18-methylicosanoic acid and other fatty acids (such as stearic, palmitic or oleic acid), cholesterol and ceramides. This may result in the loss of cuticle cells and breakage of or cracks in the hair fiber.

The exposure of hair fibers to the above environmental stress factors may lead e.g. to the loss of amino acids (Tyr, Lys, His, Pro, Val, Cys), the loss of 18-methylicosanoic acid, the degradation of melanin (particularly through excessive sun exposure), the fading of color, less brightness of the hair fiber, cuticle cell lifting and difficulties with combing the hair.

It is thus particularly advantageous to decrease the fiber breakage of human hair according to the present invention after exposure to said stress factors.

Another preferred embodiment according to the invention is a use as described herein, wherein the composition is a hair care product, more preferably is a shampoo (including anti-dandruff shampoo, baby shampoo, shampoo for dry scalps, concentrated shampoo, dry formulation of a shampoo), conditioner, preferably a leave-on or leave-in conditioner (or a dry formulation of a conditioner or derivative thereof), 2-in-1 product (comprising shampoo and conditioner), tip repairing product, hair treatment cure, hair tonic, hair lotion, hair rinse, styling cream, hair setting composition, preferably formulated as gel, mousse or spray, styling aid, blonding composition or hair coloring composition, most preferably is a shampoo, conditioner, preferably a leave-on or leave-in conditioner, or a 2-in-1 product.

Preferred hair care compositions as described herein are selected from the group of products for treatment, protecting, care and cleansing of the hair, preferably as a leave-on product (meaning that the one or more, preferably all, components of the compositions as described herein stay on the hair for a longer period of time, compared to rinse-off products, so that the effects as described above are more pronounced).

The compositions as described herein are preferably in the form of an emulsion, e.g. W/O (water-in-oil), O/W (oil-in-water), W/O/W (water-in-oil-in-water), O/W/O (oil-in-water-in-oil) emulsion, PIT emulsion, Pickering emulsion, emulsion with a low oil content, micro- or nanoemulsion, a gel (including hydrogel, hydrodispersion gel, oleogel), a solution e.g. in oil (fatty oils or fatty acid esters, in particular C₆-C₃₂ fatty acid C₂-C₃₀ esters) or silicone oil, dispersion, suspension, cream, lotion or milk, depending on the production method and ingredients, pomade, spray (e.g. pump spray or spray with propellant) or a foam, paste, oil, balsam, serum, powder, mask or aerosol (foaming, non-foaming or post-foaming).

The compositions used according to the present invention may further contain antidandruff agents, irritation-preventing agents, irritation-inhibiting agents, antioxidants, astringents, antiseptic agents, ant-statics, binders, buffers, carrier materials, chelating agents, cell stimulants, cleansing agents, care agents, softeners, emulsifiers, enzymes, essential oils, fibres, film-forming agents, fixatives, foam-forming agents, foam stabilizers, substances for preventing foaming, foam boosters, gelling agents, gel-forming agents, hair care agents, hair-setting agents, hair-straightening agents, moisture-donating agents, moisturizing substances, moisture-retaining substances, bleaching agents, strengthening agents, optically brightening agents, gloss agents, polymers, powders, proteins, re-oiling agents, silicones, hair promotion agents, cooling agents, skin-cooling agents, warming agents, skin-warming agents, stabilizers, UV-absorbing agents, UV filters, detergents, thickeners, vitamins, oils, waxes, fats, phospholipids, saturated fatty acids, mono- or polyunsaturated fatty acids, α-hydroxy acids, polyhydroxyfatty acids, liquefiers, dyestuffs, color-protecting agents, pigments, odoriferous substances, polyols, surfactants, electrolytes, organic solvents or silicone derivatives and the like as additional auxiliaries and/or additives.

### SURFACTANS

Preferred auxiliaries and/or additives are anionic and/or amphoteric and/or zwitterionic surfactants. Typical examples of anionic surfactants are soaps, alkyl benzenesulfonates, alkanesulfonates, olefin sulfonates, alkylether sulfonates, glycerol ether sulfonates, methyl ester sulfonates, sulfofatty acids, alkyl sulfates, fatty alcohol ether sulfates, glycerol ether sulfates, fatty acid ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, ether carboxylic acids and salts thereof, fatty acid isethionates, fatty acid sarcosinates, fatty acid taurides, N-acylamino acids such as, for example, acyl lactylates, acyl tartrates, acyl glutamates and acyl aspartates, alkyl oligoglucoside sulfates, protein fatty acid condensates (particularly wheat-based vegetable products) and alkyl (ether) phosphates. If the anionic surfactants contain polyglycol ether chains, they may have a conventional homolog distribution although they preferably have a narrow-range homolog distribution. Typical examples of amphoteric or zwitterionic surfactants are alkylbetaines, alkylamidobetaines, aminopropionates, aminoglycinates, imidazolinium betaines and sulfobetaines. The surfactants mentioned are all known compounds. Information on their structure and production can be found in relevant synoptic works, cf. for example J. Falbe (ed.), "Surfactants in Consumer Products", Springer Verlag, Berlin, 1987, pages 54 to 124 or J. Falbe (ed.), "Katalysatoren, Tenside und Mineralöladditive (Catalysts, Surfactants and Mineral Oil Additives)", Thieme Verlag, Stuttgart, 1978, pages 123-217. The percentage content of surfactants in the compositions may be from 0.1 to 10% by weight and is preferably from 0.5 to 5% by weight, based on the total weight of the composition.

### OIL BODIES

Suitable oil bodies, which form constituents of O/W emulsions, are, for example, Guerbet alcohols based on fatty alcohols having 6 to 18, preferably 8 to 10, carbon atoms, esters of linear C₆-C₂₂-fatty acids with linear or branched C₆-C₂₂-fatty alcohols or esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. Also suitable are esters of linear C₆-C₂₂-fatty acids with branched alcohols, in particular 2-ethylhexanol, esters of C₁₈-C₃₈-alkylhydroxy carboxylic acids with linear or branched C₆-C₂₂-fatty alcohols, in particular dioctyl malate, esters of linear and/or branched fatty acids with polyhydric alcohols (such as, for example, propylene glycol, dimerdiol or trimertriol) and/or Guerbet alcohols, triglycerides based on C₆-C₁₀-fatty acids, liquid mono-/di-/triglyceride mixtures based on C₆-C₁₈-fatty acids, esters of C₆- C₂₂-fatty alcohols and/or Guerbet alcohols with aromatic carboxylic acids, in particular benzoic acid, esters of C₂-C₁₂-dicarboxylic acids with linear or branched alcohols having 1 to 22 carbon atoms or polyols having 2 to 10 carbon atoms and 2 to 6 hydroxyl groups, vegetable oils, branched primary alcohols, substituted cyclohexanes, linear and branched C₆-C₂₂-fatty alcohol carbonates, such as, for example, Dicaprylyl Carbonate (Cetiol^{®} CC), Guerbet carbonates, based on fatty alcohols having 6 to 18, preferably 8 to 10, carbon atoms, esters of benzoic acid with linear and/or branched C₆-C₂₂-alcohols (e.g. Finsolv^{®} TN), linear or branched, symmetrical or asymmetrical dialkyl ethers having 6 to 22 carbon atoms per alkyl group, such as, for example, dicaprylyl ether (Cetiol^{®} OE), ring-opening products of epoxidized fatty acid esters with polyols, silicone oils (cyclomethicones, silicone methicone grades, etc.) and/or aliphatic or naphthenic hydrocarbons, such as, for example, squalane, squalene or dialkylcyclohexanes.

### EMULSIFIERS

Other surfactants may also be added to the compositions as emulsifiers, including for example:
- Products of the addition of 2 to 30 mol ethylene oxide and/or 0 to 5 mol propylene oxide onto linear C₈₋₂₂ fatty alcohols, onto C₁₂₋₂₂ fatty acids and onto alkyl phenols containing 8 to 15 carbon atoms in the alkyl group;
- C_{12/18} fatty acid monoesters and diesters of addition products of 1 to 30 mol ethylene oxide onto glycerol;
- glycerol mono- and diesters and sorbitan mono- and diesters of saturated and unsaturated fatty acids containing 6 to 22 carbon atoms and ethylene oxide addition products thereof;
- addition products of 15 to 60 mol ethylene oxide onto castor oil and/or hydrogenated castor oil;
- polyol esters and, in particular, polyglycerol esters such as, for example, polyglycerol polyricinoleate, polyglycerol poly-12-hydroxystearate or polyglycerol dimerate isostearate. Mixtures of compounds from several of these classes are also suitable;
- addition products of 2 to 15 mol ethylene oxide onto castor oil and/or hydrogenated castor oil;
- partial esters based on linear, branched, unsaturated or saturated C_{6/22} fatty acids, ricinoleic acid and 12-hydroxystearic acid and glycerol, polyglycerol, pentaerythritol, dipentaerythritol, sugar alcohols (for example sorbitol), alkyl glucosides (for example methyl glucoside, butyl glucoside, lauryl glucoside) and polyglucosides (for example cellulose);
- mono-, di and trialkyl phosphates and mono-, di- and/or tri-PEG-alkyl phosphates and salts thereof;
- wool wax alcohols;
- polysiloxane/polyalkyl polyether copolymers and corresponding derivatives;
- mixed esters of pentaerythritol, fatty acids, citric acid and fatty alcohol and/or mixed esters of C₆₋₂₂ fatty acids, methyl glucose and polyols, preferably glycerol or polyglycerol,
- polyalkylene glycols and
- glycerol carbonate.

The addition products of ethylene oxide and/or propylene oxide onto fatty alcohols, fatty acids, alkylphenols, glycerol mono- and diesters and sorbitan mono- and diesters of fatty acids or onto castor oil are known commercially available products. They are homologue mixtures of which the average degree of alkoxylation corresponds to the ratio between the quantities of ethylene oxide and/or propylene oxide and substrate with which the addition reaction is carried out. C_{12/18} fatty acid monoesters and diesters of addition products of ethylene oxide onto glycerol are known as lipid layer enhancers for cosmetic formulations. The preferred emulsifiers are described in more detail as follows:
**Partial glycerides.** Typical examples of suitable partial glycerides are hydroxystearic acid monoglyceride, hydroxystearic acid diglyceride, isostearic acid monoglyceride, isostearic acid diglyceride, oleic acid monoglyceride, oleic acid diglyceride, ricinoleic acid monoglyceride, ricinoleic acid diglyceride, linoleic acid monoglyceride, linoleic acid diglyceride, linolenic acid monoglyceride, linolenic acid diglyceride, erucic acid monoglyceride, erucic acid diglyceride, tartaric acid monoglyceride, tartaric acid diglyceride, citric acid monoglyceride, citric acid diglyceride, malic acid monoglyceride, malic acid diglyceride and technical mixtures thereof which may still contain small quantities of triglyceride from the production process. Addition products of 1 to 30 and preferably 5 to 10 mol ethylene oxide onto the partial glycerides mentioned are also suitable.

**Sorbitan esters.** Suitable sorbitan esters are sorbitan monoisostearate, sorbitan sesqui-isostearate, sorbitan diisostearate, sorbitan triisostearate, sorbitan monooleate, sorbitan sesquioleate, sorbitan dioleate, sorbitan trioleate, sorbitan monoerucate, sorbitan sesquierucate, sorbitan dierucate, sorbitan trierucate, sorbitan monoricinoleate, sorbitan sesquiricinoleate, sorbitan diricinoleate, sorbitan triricinoleate, sorbitan monohydroxystearate, sorbitan sesquihydroxystearate, sorbitan dihydroxystearate, sorbitan trihydroxystearate, sorbitan monotartrate, sorbitan sesquitartrate, sorbitan ditartrate, sorbitan tritartrate, sorbitan monocitrate, sorbitan sesquicitrate, sorbitan dicitrate, sorbitan tricitrate, sorbitan monomaleate, sorbitan sesquimaleate, sorbitan dimaleate, sorbitan trimaleate and technical mixtures thereof. Addition products of 1 to 30 and preferably 5 to 10 mol ethylene oxide onto the sorbitan esters mentioned are also suitable.

**Polyglycerol esters.** Typical examples of suitable polyglycerol esters are Polyglyceryl-2 Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Beilina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) and Polyglyceryl Polyricinoleate (Admul^{®} WOL 1403), Polyglyceryl Dimerate Isostearate and mixtures thereof. Examples of other suitable polyolesters are the mono-, di- and triesters of trimethylol propane or pentaerythritol with lauric acid, cocofatty acid, tallow fatty acid, palmitic acid, stearic acid, oleic acid, behenic acid and the like optionally reacted with 1 to 30 mol ethylene oxide.

**Anionic emulsifiers.** Typical anionic emulsifiers are aliphatic C₁₂₋₂₂ fatty acids, such as palmitic acid, stearic acid or behenic acid for example, and C₁₂₋₂₂ dicarboxylic acids, such as azelaic acid or sebacic acid for example.

**Amphoteric emulsifiers.** Other suitable emulsifiers are amphoteric or zwitterionic surfactants. Zwitterionic surfactants are surface-active compounds which contain at least one quaternary ammonium group and at least one carboxylate and one sulfonate group in the molecule. Particularly suitable zwitterionic surfactants are the so-called betaines, such as the N-alkyl-N,N-dimethyl ammonium glycinates, for example cocoalkyl dimethyl ammonium glycinate, N-acylaminopropyl-N,N-dimethyl ammonium glycinates, for example cocoacylaminopropyl dimethyl ammonium glycinate, and 2-alkyl-3-carboxymethyl-3-hydroxyethyl imidazolines containing 8 to 18 carbon atoms in the alkyl or acyl group and cocoacylaminoethyl hydroxyethyl carboxymethyl glycinate. The fatty acid amide derivative known under the CTFA name of *Cocamidopropyl Betaine* is particularly preferred. Ampholytic surfactants are also suitable emulsifiers. Ampholytic surfactants are surface-active compounds which, in addition to a C_{8/18} alkyl or acyl group, contain at least one free amino group and at least one -COOH- or -SOsH- group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalkylaminopropionate, cocoacylaminoethyl aminopropionate and C_{12/18} acyl sarcosine.

### SUPERFATTING AGENTS AND CONSISTENCY FACTORS

Superfatting agents may be selected from such substances as, for example, lanolin and lecithin and also polyethoxylated or acylated lanolin and lecithin derivatives, polyol fatty acid esters, monoglycerides and fatty acid alkanolamides, the fatty acid alkanolamides also serving as foam stabilizers.

The consistency factors mainly used are fatty alcohols or hydroxyfatty alcohols containing 12 to 22 and preferably 16 to 18 carbon atoms and also partial glycerides, fatty acids or hydroxyfatty acids. A combination of these substances with alkyl oligoglucosides and/or fatty acid N-methyl glucamides of the same chain length and/or polyglycerol poly-12-hydroxystearates is preferably used.

### THICKENING AGENTS AND RHEOLOGY ADDITIVES

Suitable thickeners are polymeric thickeners, such as Aerosil^{®} types (hydrophilic silicas), polysaccharides, more especially xanthan gum, guar-guar, agar-agar, alginates and tyloses, carboxymethyl cellulose and hydroxyethyl cellulose, also relatively high molecular weight polyethylene glycol monoesters and diesters of fatty acids, polyacrylates (for example Carbopols^{®} [Goodrich] or Synthalens^{®} [Sigma]), polyacrylamides, polyvinyl alcohol and polyvinyl pyrrolidone, surfactants such as, for example, ethoxylated fatty acid glycerides, esters of fatty acids with polyols, for example pentaerythritol or trimethylol propane, narrow-range fatty alcohol ethoxylates and electrolytes, such as sodium chloride and ammonium chloride.

### POLYMERS

Suitable cationic polymers are, for example, cationic cellulose derivatives such as, for example, the quaternized hydroxyethyl cellulose obtainable from Amerchol under the name of Polymer JR 400^{®}, cationic starch, copolymers of diallyl ammonium salts and acrylamides, quaternized vinyl pyrrolidone/vinyl imidazole polymers such as, for example, Luviquat^{®} (BASF), condensation products of polyglycols and amines, quaternized collagen polypeptides such as, for example, Lauryldimonium Hydroxypropyl Hydrolyzed Collagen (Lamequat^{®} L, Grünau), quaternized wheat polypeptides, polyethyleneimine, cationic silicone polymers such as, for example, amodimethicone, copolymers of adipic acid and dimethylaminohydroxypropyl diethylenetriamine (Cartaretine^{®}, Sandoz), copolymers of acrylic acid with dimethyl diallyl ammonium chloride (Merquat^{®} 550, Chemviron), polyaminopolyamides and crosslinked water-soluble polymers thereof, cationic chitin derivatives such as, for example, quaternized chitosan, optionally in microcrystalline distribution, condensation products of dihaloalkyls, for example dibromobutane, with bis-dialkylamines, for example bis-dimethylamino-1 ,3-propane, cationic guar gum such as, for example, Jaguar^{®}CBS, Jaguar^{®}C-17, Jaguar^{®}C-16 of Celanese, quaternized ammonium salt polymers such as, for example, Mirapol^{®} A-15, Mirapol^{®} AD-1, Mirapol^{®} AZ-1 of Miranol and the various polyquaternium types (for example 6, 7, 32 or 37) which can be found in the market under the tradenames Rheocare^{®} CC or Ultragel^{®} 300.

Suitable anionic, zwitterionic, amphoteric and nonionic polymers are, for example, vinyl acetate/crotonic acid copolymers, vinyl pyrrolidone/vinyl acrylate copolymers, vinyl acetate/butyl maleate/isobornyl acrylate copolymers, methyl vinylether/maleic anhydride copolymers and esters thereof, uncrosslinked and polyol-crosslinked polyacrylic acids, acrylamidopropyl trimethylammonium chloride/acrylate copolymers, octylacrylamide/methyl methacrylate/tert.-butylaminoethyl methacrylate/2-hydroxypropyl methacrylate copolymers, polyvinyl pyrrolidone, vinyl pyrrolidone/vinyl acetate copolymers, vinyl pyrrolidone/dimethylaminoethyl methacrylate/vinyl caprolactam terpolymers and optionally derivatized cellulose ethers and silicones.

### PEARLIZING WAXES

Suitable pearlising waxes are, for example, alkylene glycol esters, especially ethylene glycol distearate; fatty acid alkanolamides, especially cocofatty acid diethanolamide; partial glycerides, especially stearic acid monoglyceride; esters of polybasic, optionally hydroxysubstituted carboxylic acids with fatty alcohols containing 6 to 22 carbon atoms, especially long-chain esters of tartaric acid; fatty compounds, such as for example fatty alcohols, fatty ketones, fatty aldehydes, fatty ethers and fatty carbonates which contain in all at least 24 carbon atoms, especially laurone and distearylether; fatty acids, such as stearic acid, hydroxystearic acid or behenic acid, ring opening products of olefin epoxides containing 12 to 22 carbon atoms with fatty alcohols containing 12 to 22 carbon atoms and/or polyols containing 2 to 15 carbon atoms and 2 to 10 hydroxyl groups and mixtures thereof.

### SILICONES

Suitable silicone compounds are, for example, dimethyl polysiloxanes, methylphenyl polysiloxanes, cyclic silicones and amino-, fatty acid-, alcohol-, polyether-, epoxy-, fluorine-, glycoside- and/or alkyl-modified silicone compounds which may be both liquid and resin-like at room temperature. Other suitable silicone compounds are simethicones which are mixtures of dimethicones with an average chain length of 200 to 300 dimethylsiloxane units and hydrogenated silicates. A detailed overview of suitable volatile silicones can be found in Todd et al. in Cosm. Toil. 91, 27 (1976).

### WAXES AND STABILIZERS

Besides natural oils, waxes may also be present in the compositions, more especially natural waxes such as, for example, candelilla wax, carnauba wax, Japan wax, espartograss wax, cork wax, guaruma wax, rice oil wax, sugar cane wax, ouricury wax, montan wax, beeswax, shellac wax, spermaceti, lanolin (wool wax), uropygial fat, ceresine, ozocerite (earth wax), petrolatum, paraffin waxes and microwaxes; chemically modified waxes (hard waxes) such as, for example, montan ester waxes, sasol waxes, hydrogenated jojoba waxes and synthetic waxes such as, for example, polyalkylene waxes and polyethylene glycol waxes.

Metal salts of fatty acids such as, for example, magnesium, aluminium and/or zinc stearate or ricinoleate may be used as stabilizers.

### PRIMARY SUN PROTECTION FACTORS

Primary sun protection factors in the context of the present invention are, for example, organic substances (light filters) which are liquid or crystalline at room temperature and which are capable of absorbing ultraviolet radiation and of releasing the energy absorbed in the form of longer-wave radiation, for example heat.

The compositions used according to the invention advantageously contain at least one UV-A filter and/or at least one UV-B filter and/or a broadband filter and/or at least one inorganic pigment. Formulations used according to the invention preferably contain at least one UV-B filter or a broadband filter, more particularly preferably at least one UV-A filter and at least one UV-B filter.

Preferred cosmetic compositions comprise one, two, three or more sun protection factors selected from the group consistiung of 4-aminobenzoic acid and derivatives, salicylic acid derivatives, benzophenone derivatives, dibenzoylmethane derivatives, diphenyl acrylates, 3-imidazol-4-yl acrylic acid and esters thereof, benzofuran derivatives, benzylidene malonate derivatives, polymeric UV absorbers containing one or more organosilicon radicals, cinnamic acid derivatives, camphor derivatives, trianilino-s-triazine derivatives, 2-hydroxyphenylbenzotriazole derivatives, phenylbenzimidazole sulfonic acid derivatives and salts thereof, anthranilic acid menthyl esters, benzotriazole derivatives and indole derivatives.

The UV filters cited below, which can be used within the context of the present invention, are preferred but naturally are not limiting.

UV filters which are preferably used are selected from the group consisting of
- p-aminobenzoic acid
- p-aminobenzoic acid ethyl ester (25 mol) ethoxylated (INCI name: PEG-25 PABA)
- p-dimethylaminobenzoic acid-2-ethylhexyl ester
- p-aminobenzoic acid ethyl ester (2 mol) N-propoxylated
- p-aminobenzoic acid glycerol ester
- salicylic acid homomenthyl ester (homosalates) (Neo Heliopan°HMS)
- salicylic acid-2-ethylhexyl ester (Neo Heliopan^{®}OS)
- triethanolamine salicylate
- 4-isopropyl benzyl salicylate
- anthranilic acid menthyl ester (Neo Heliopan^{®}MA)
- diisopropyl cinnamic acid ethyl ester
- p-methoxycinnamic acid-2-ethylhexyl ester (Neo Heliopan^{®}AV)
- diisopropyl cinnamic acid methyl ester
- p-methoxycinnamic acid isoamyl ester (Neo Heliopan^{®}E 1000)
- p-methoxycinnamic acid diethanolamine salt
- p-methoxycinnamic acid isopropyl ester
- 2-phenylbenzimidazole sulfonic acid and salts (Neo Heliopan^{®}Hydro)
- 3-(4'-trimethylammonium) benzylidene bornan-2-one methyl sulfate
- beta-imidazole-4(5)-acrylic acid (urocanic acid)
- 3-(4'-sulfo)benzylidene bornan-2-one and salts
- 3-(4'-methyl benzylidene)-D,L-camphor (Neo Heliopan^{®}MBC)
- 3-benzylidene-D,L-camphor
- N-[(2 and 4)-[2-(oxoborn-3-ylidene) methyl]benzyl] acrylamide polymer
- 4,4'-[(6-[4-(1,1-dimethyl)aminocarbonyl)phenylamino]-1,3,5-triazine-2,4-diyl)diimino]-bis-(benzoic acid-2-ethylhexyl ester) (Uvasorb^{®}HEB)
- benzylidene malonate polysiloxane (Parsol^{®}SLX)
- glyceryl ethylhexanoate dimethoxycinnamate
- dipropylene glycol salicylate
- tris(2-ethylhexyl)-4,4',4"-(1,3,5-triazine-2,4,6-triyltriimino)tribenzoate (= 2,4,6-trianilino-(p-carbo-2'-ethylhexyl-1 '-oxy)-1 ,3,5-triazine) (Uvinul^{®}T150).

Broadband filters which are preferably contained in the compositions described herein are selected from the group consisting of
- 2-ethylhexyl-2-cyano-3,3-diphenyl acrylate (Neo Heliopan^{®}303)
- ethyl-2-cyano-3,3'-diphenyl acrylate
- 2-hydroxy-4-methoxybenzophenone (Neo Heliopan^{®}BB)
- 2-hydroxy-4-methoxybenzophenone-5-sulfonic acid
- dihydroxy-4-methoxybenzophenone
- 2,4-dihydroxybenzophenone
- tetrahydroxybenzophenone
- 2,2'-dihydroxy-4,4'-dimethoxybenzophenone
- 2-hydroxy-4-n-octoxybenzophenone
- 2-hydroxy-4-methoxy-4'-methyl benzophenone
- sodium hydroxymethoxybenzophenone sulfonate
- disodium-2,2'-dihydroxy-4,4'-dimethoxy-5,5'-disulfobenzophenone
- phenol, 2-(2H-benzotriazol-2-yl)-4-methyl-6-(2-methyl-3(1,3,3,3-tetramethyl-1-(trime-thylsilyl)oxy)disiloxyanyl) propyl) (MexorylOXL)
- 2,2'-methylene bis-(6-(2H-benzotriazol-2-yl)-4-1,1,3,3-tetramethylbutyl) phenol) (Tinosorb^{®}M)
- 2,4-bis-[4-(2-ethylhexyloxy)-2-hydroxyphenyl]-1,3,5-triazine
- 2,4-bis-[{(4-(2-ethylhexyloxy)-2-hydroxy}phenyl]-6-(4-methoxyphenyl)-1,3,5-triazine (Tinosorb^{®}S)
- 2,4-bis-[{(4-(3-sulfonato)-2-hydroxypropyloxy)-2-hydroxy}phenyl]-6-(4-methoxyphenyl)-1,3,5-triazine sodium salt
- 2,4-bis-[{(3-(2-propyloxy)-2-hydroxypropyloxy)-2-hydroxy}phenyl]-6-(4-methoxyphenyl)-1,3,5-triazine
- 2,4-bis-[{4-(2-ethylhexyloxy)-2-hydroxy}phenyl]-6-[4-(2-methoxyethyl carbonyl) phenylamino]-1,3,5-triazine
- 2,4-bis-[{4-(3-(2-propyloxy)-2-hydroxypropyloxy)-2-hydroxy}phenyl]-6-[4-(2-ethylcarboxyl) phenylamino]-1,3,5-triazine
- 2,4-bis-[{4-(2-ethylhexyloxy)-2-hydroxy}phenyl]-6-(1-methylpyrrol-2-yl)-1,3,5-triazine
- 2,4-bis-[{4-tris-(trimethylsiloxysilylpropyloxy)-2-hydroxy}phenyl]-6-(4-methoxyphenyl)-1,3,5-triazine
- 2,4-bis-[{4-(2"-methylpropenyloxy)-2-hydroxy}phenyl]-6-(4-methoxyphenyl)-1,3,5-triazine
- 2,4-bis-[{4-(1',1',1',3',5',5',5'-heptamethylsiloxy-2"-methylpropyloxy)-2-hydroxy}phenyl]-6-(4-methoxyphenyl)-1,3,5-triazine.

The compositions can comprise further typical detergent and cleansing composition ingredients such as UV-A filters, which are preferably selected from the group consisting of
- 4-isopropyl dibenzoyl methane
- terephthalylidene dibornane sulfonic acid and salts (Mexoryl^{®}SX)
- 4-t-butyl-4'-methoxydibenzoyl methane (avobenzone) / (Neo Heliopan^{®}357)
- phenylene bis-benzimidazyl tetrasulfonic acid disodium salt (Neo Heliopan^{®}AP)
- 2,2'-(1,4-phenylene)-bis-(1H-benzimidazole-4,6-disulfonic acid), monosodium salt
- 2-(4-diethylamino-2-hydroxybenzoyl) benzoic acid hexyl ester (Uvinul^{®} A Plus)
- indanylidene compounds in accordance with DE 100 55 940 A1 (= WO 2002 038537 A1)

The compositions can comprise further typical detergent and cleansing composition ingredients such as UV filters which are preferably selected from the group consisting of
- p-aminobenzoic acid
- 3-(4'-trimethylammonium) benzylidene bornan-2-one methyl sulfate
- salicylic acid homomenthyl ester (Neo Heliopan^{®}HMS)
- 2-hydroxy-4-methoxybenzophenone (Neo Heliopan^{®}BB)
- 2-phenylbenzimidazole sulfonic acid (Neo Heliopan^{®}Hydro)
- terephthalylidene dibornane sulfonic acid and salts (Mexoryl^{®}SX)
- 4-tert-butyl-4'-methoxydibenzoyl methane (Neo Heliopan^{®}357)
- 3-(4'-sulfo)benzylidene bornan-2-one and salts
- 2-ethylhexyl-2-cyano-3,3-diphenyl acrylate (Neo Heliopan^{®}303)
- N-[(2 and 4)-[2-(oxoborn-3-ylidene) methyl]benzyl] acrylamide polymer
- p-methoxycinnamic acid-2-ethylhexyl ester (Neo Heliopan^{®}AV)
- p-aminobenzoic acid ethyl ester (25 mol) ethoxylated (INCI name: PEG-25 PABA)
- p-methoxycinnamic acid isoamyl ester (Neo Heliopan^{®}E1000)
- 2,4,6-trianilino-(p-carbo-2'-ethylhexyl-1'-oxy)-1,3,5-triazine (Uvinul^{®}T150)
- phenol,2-(2H-benzotriazol-2-yl)-4-methyl-6-(2-methyl-3(1,3,3,3-tetramethyl-1-(trimethylsilyl)oxy)disiloxyanyl) propyl) (Mexoryl^{®}XL)
- 4,4'-[(6-[4-(1,1-dimethyl)aminocarbonyl)phenylamino]-1,3,5-triazine-2,4-diyl)diimino]-bis-(benzoic acid-2-ethylhexyl ester) (Uvasorb HEB)
- 3-(4'-methyl benzylidene)-D,L-camphor (Neo Heliopan^{®}MBC)
- 3-benzylidene camphor
- salicylic acid-2-ethylhexyl ester (Neo Heliopan^{®}OS)
- 4-dimethylaminobenzoic acid-2-ethylhexyl ester (Padimate O)
- hydroxy-4-methoxybenzophenone-5-sulfonic acid and Na salt
- 2,2'-methylene bis-(6-(2H-benzotriazol-2-yl)-4-1,1,3,3-tetramethylbutyl) phenol) (Tinosorb^{®}M)
- phenylene bis-benzimidazyl tetrasulfonic acid disodium salt (Neo Heliopan^{®}AP)
- 2,4-bis-[{(4-(2-ethylhexyloxy)-2-hydroxy}phenyl]-6-(4-methoxyphenyl)-1,3,5-triazine (Tinosorb^{®}S)
- benzylidene malonate polysiloxane (Parsol^{®}SLX)
- menthyl anthranilate (Neo Heliopan^{®}MA)
- 2-(4-diethylamino-2-hydroxybenzoyl) benzoic acid hexyl ester (Uvinul^{®} A Plus)
- indanylidene compounds in accordance with DE 100 55 940 (= WO 02/38537).

Further advantageous primary and also secondary sun protection factors are mentioned in WO 2005 123101 A1. Advantageously, these preparations contain at least one UVA filter and/or at least one UVB filter and/or at least one inorganic pigment. The preparations may be present here in various forms such as are conventionally used for sun protection preparations. Thus, they may be in form of a solution, an emulsion of the water-in-oil type (W/O) or of the oil-in-water type (O/W) or a multiple emulsion, for example of the water-in-oil-in-water type (W/O/W), a gel, a hydrodispersion, a solid stick or else an aerosol.

Preferably a composition as described herein contains a total amount of sunscreen agents, i.e. in particular UV filters and/or inorganic pigments (UV filtering pigments) so that the composition has a light protection factor of greater than or equal to 2 (preferably greater than or equal to 5). Such compositions are particularly suitable for protecting the hair fiber of human hair.

### SECONDARY SUN PROTECTION FACTORS

Besides the groups of primary sun protection factors mentioned above, secondary sun protection factors of the antioxidant type may also be used. Secondary sun protection factors of the antioxidant type interrupt the photochemical reaction chain which is initiated when UV rays penetrate into the hair. Typical examples are amino acids (for example glycine, histidine, tyrosine, tryptophane) and derivatives thereof, imidazoles (for example urocanic acid) and derivatives thereof, peptides, such as D,L-carnosine, D-carnosine, L-carnosine and derivatives thereof (for example anserine), carotinoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxine, glutathione, cysteine, cystine, cystamine and glycosyl, N-acetyl, methyl, ethyl, propyl, amyl, butyl and lauryl, palmitoyl, oleyl, alpha-linoleyl, cholesteryl and glyceryl esters thereof) and their salts, dilaurylthiodipropionate, distearylthiodipropionate, thiodipropionic acid and derivatives thereof (esters, ethers, peptides, lipids, nucleotides, nucleosides and salts) and sulfoximine compounds (for example butionine sulfoximines, homocysteine sulfoximine, butionine sulfones, penta-, hexa- and hepta-thionine sulfoximine) in very small compatible dosages, also (metal) chelators (for example alpha-hydroxyfatty acids, palmitic acid, phytic acid, lactoferrine), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example linoleic acid, oleic acid), folic acid and derivatives thereof, ubiquinone and ubiquinol and derivatives thereof, vitamin C and derivatives thereof (for example ascorbyl palmitate, Mg ascorbyl phosphate, ascorbyl acetate), tocopherols and derivatives (for example vitamin E acetate), vitamin A and derivatives (vitamin A palmitate) and coniferyl benzoate of benzoin resin, rutinic acid and derivatives thereof, glycosyl rutin, ferulic acid, furfurylidene glucitol, carnosine, butyl hydroxytoluene, butyl hydroxyanisole, nordihydroguaiac resin acid, nordihydroguaiaretic acid, trihydroxybutyrophenone, uric acid and derivatives thereof, mannose and derivatives thereof, superoxide dismutase, titanium dioxide (for example dispersions in ethanol), zinc and derivatives thereof (for example ZnO, ZnSO₄), selenium and derivatives thereof (for example selenium methionine), stilbenes and derivatives thereof (for example stilbene oxide, trans-stilbene oxide) and derivatives of these active substances suitable for the purposes of the invention (salts, esters, ethers, sugars, nucleotides, nucleosides, peptides and lipids).

Advantageous inorganic secondary light protection pigments are finely dispersed metal oxides and metal salts which are also mentioned in WO 2005 123101 A1. The total quantity of inorganic pigments, in particular hydrophobic inorganic micro-pigments in a finished cosmetic composition is advantageously from 0.1 to 30% by weight, preferably 0.5 to 10.0% by weight, in each case based on the total weight of the composition.

Also preferred are particulate UV filters or inorganic pigments, which can optionally be hydrophobed, such as the oxides of titanium (TiO₂), zinc (ZnO), iron (Fe₂O₃), zirconium (ZrO₂), silicon (SiO₂), manganese (e.g. MnO), aluminium (Al₂O₃), cerium (e.g. Ce₂O₃) and/or mixtures thereof.

### ACTIVES MODULATING HAIR PIGMENTATION

Preferred active ingredients for hair lightening are selected from the group consisting of: kojic acid (5-hydroxy-2-hydroxymethyl-4-pyranone), kojic acid derivatives, preferably kojic acid dipalmitate, arbutin, ascorbic acid, ascorbic acid derivatives, preferably magnesium ascorbyl phosphate, hydroquinone, hydroquinone derivatives, resorcinol, resorcinol derivatives, preferably 4-alkylresorcinols and 4-(1-phenylethyl)1,3-dihydroxybenzene (phenylethyl resorcinol), cyclohexylcarbamates (preferably one or more cyclohexyl carbamates disclosed in WO 2010/122178 and WO 2010/097480), sulfur-containing molecules, preferably glutathione or cysteine, alpha-hydroxy acids (preferably citric acid, lactic acid, malic acid), salts and esters thereof, N-acetyl tyrosine and derivatives, undecenoyl phenylalanine, gluconic acid, chromone derivatives, preferably aloesin, flavonoids, 1-aminoethyl phosphinic acid, thiourea derivatives, ellagic acid, nicotinamide (niacinamide), zinc salts, preferably zinc chloride or zinc gluconate, thujaplicin and derivatives, triterpenes, preferably maslinic acid, sterols, preferably ergosterol, benzofuranones, preferably senkyunolide, vinyl guiacol, ethyl guiacol, dionic acids, preferably octodecene dionic acid and/or azelaic acid, inhibitors of nitrogen oxide synthesis, preferably L-nitroarginine and derivatives thereof, 2,7-dinitroindazole or thiocitrulline, metal chelators (preferably alpha-hydroxy fatty acids, phytic acid, humic acid, bile acid, bile extracts, EDTA, EGTA and derivatives thereof), retinoids, soy milk and extract, serine protease inhibitors or lipoic acid or other synthetic or natural active ingredients for hair lightening, the latter preferably used in the form of an extract from plants, preferably bearberry extract, rice extract, papaya extract, turmeric extract, mulberry extract, bengkoang extract, nutgrass extract, liquorice root extract or constituents concentrated or isolated therefrom, preferably glabridin or licochalcone A, artocarpus extract, extract of rumex and ramulus species, extracts of pine species (pinus), extracts of vitis species or stilbene derivatives isolated or concentrated therefrom, saxifrage extract, scutelleria extract, grape extract and/or microalgae extract, in particular Tetraselmis suecica Extract.

Advantageous hair tanning active ingredients in this respect are substrates or substrate analogues of tyrosinase such as L-tyrosine, N-acetyl tyrosine, L-DOPA or L-dihydroxyphenylalanine, xanthine alkaloids such as caffeine, theobromine and theophylline and derivatives thereof, proopiomelanocortin peptides such as ACTH, alpha-MSH, peptide analogues thereof and other substances which bind to the melanocortin receptor, peptides such as Val-Gly-Val-Ala-Pro-Gly, Lys-Ile- Gly-Arg-Lys or Leu-Ile-Gly-Lys, purines, pyrimidines, folic acid, copper salts such as copper gluconate, chloride or pyrrolidonate, 1,3,4-oxadiazole-2-thiols such as 5-pyrazin-2-yl-1,3,4-oxadiazole-2-thiol, curcumin, zinc diglycinate (Zn(Gly)2), manganese(II) bicarbonate complexes ("pseudocat-alases") as described for example in EP 0 584 178, tetrasubstituted cyclohexene deriva-tives as described for example in WO 2005/032501, isoprenoids as described in WO 2005/102252 and in WO 2006/010661, melanin derivatives such as Melasyn-100 and MelanZe, diacyl glycerols, aliphatic or cyclic diols, psoralens, prostaglandins and ana-logues thereof, activators of adenylate cyclase and compounds which activate the transfer of melanosomes to keratinocytes such as serine proteases or agonists of the PAR-2 receptor, extracts of plants and plant parts of the chrysanthemum species, san-guisorba species, walnut extracts, urucum extracts, rhubarb extracts, trehalose, erythrulose and dihydroxyacetone. Flavonoids which bring about hair tinting or browning (e.g. quercetin, rhamnetin, kaempferol, fisetin, genistein, daidzein, chrysin and apigenin, epicatechin, diosmin and diosmetin, morin, quercitrin, naringenin, hesperidin, phloridzin and phloretin) can also be used.

The amount of the aforementioned examples of additional active ingredients for the modulation of hair pigmentation (one or more compounds) in the compositions as described herein preferably is 0.00001 to 30 wt.%, preferably 0.0001 to 20 wt.%, particularly preferably 0.001 to 5 wt.%, based on the total weight of the composition.

### HAIR GROWTH ACTIVATORS OR INHIBITORS

The compositions as described herein may also comprise one or more hair growth activators, i.e. agents to stimulate hair growth. Hair growth activators are preferably selected from the group consisting of pyrimidine derivatives such as 2,4-diaminopyrimidine-3-oxide (Aminexil), 2,4-diamino-6-piperidinopyrimidine-3-oxide (Minoxidil) and derivatives thereof, 6-amino-1,2-dihydro-1-hydroxy-2-imino-4-piperidinopyrimidine and its derivatives, xanthine alkaloids such as caffeine, theobromine and theophylline and derivatives thereof, quercetin and derivatives, dihydroquercetin (taxifolin) and derivatives, potassium channel openers, antiandrogenic agents, synthetic or natural 5-reductase inhibitors, nicotinic acid esters such as tocopheryl nicotinate, benzyl nicotinate and C1-C6 alkyl nicotinate, proteins such as for example the tripeptide Lys-Pro-Val, diphencypren, hormons, finasteride, dutasteride, flutamide, bicalutamide, pregnane derivatives, progesterone and its derivatives, cyproterone acetate, spironolactone and other diuretics, calcineurin inhibitors such as FK506 (Tacrolimus, Fujimycin) and its derivatives, Cyclosporin A and derivatives thereof, zinc and zinc salts, polyphenols, procyanidins, proanthocyanidins, phytosterols such as for example beta-sitosterol, biotin, eugenol, (t)-beta-citronellol, panthenol, glycogen for example from mussels, extracts from microorganisms, algae, plants and plant parts of for example the genera dandelion (Leontodon or Taraxacum), Orthosiphon, Vitex, Coffea, Paullinia, Theobroma, Asiasarum, Cucurbita or Styphnolobium, Serenoa repens (saw palmetto), Sophora flavescens, Pygeum africanum, Panicum miliaceum, Cimicifuga racemosa, Glycine max, Eugenia caryophyllata, Cotinus coggygria, Hibiscus rosa-sinensis, Camellia sinensis, Ilex paraguariensis, licorice, grape, apple, barley or hops and/or hydrolysates from rice or wheat.

Alternatively, the compositions as described herein may comprise one or more hair growth inhibitors, i.e. agents to reduce or prevent hair growth. Hair growth inhibitors are preferably selected from the group consisting of activin, activin derivatives or activin agonists, ornithine decarboxylase inhibitors such as alpha-difluoromethylornithine or pentacyclic triterpenes like for example ursolic acid, betulin, betulinic acid, oleanolic acid and derivatives thereof, 5alpha-reductase inhibitors, androgen receptor antagonists, S-adenosylmethionine decarboxylase inhibitors, gamma-glutamyl transpeptidase inhibitors, transglutaminase inhibitors, soybean-derived serine protease inhibitors, extracts from microorganisms, algae, different microalgae or plants and plant parts of for example the families Leguminosae, Solanaceae, Graminae, Asclepiadaceae or Cucurbitaceae, the genera Chondrus, Gloiopeltis, Ceramium, Durvillea, Glycine max, Sanguisorba officinalis, Calendula officinalis, Hamamelis virginiana, Arnica montana, Salix alba, Hypericum perforatum or Gymnema sylvestre.

### PHYSIOLOGICAL COOLING AGENTS

The compositions as described herein may also contain one or more substances with a physiological cooling effect (cooling agents), which are preferably selected from the following list: menthol and menthol derivatives (for example L-menthol, D-menthol, racemic menthol, isomenthol, neoisomenthol, neomenthol) menthylethers (for example (l-menthoxy)-1,2-propandiol, (l-menthoxy)-2-methyl-1,2-propandiol, l-menthyl-methylether), menthylesters (for example menthylformiate, menthylacetate, menthylisobutyrate, menthyllactates, L-menthyl-L-lactate, L-menthyl-D-lactate, menthyl-(2-methoxy)acetate, menthyl-(2-methoxyethoxy)acetate, menthylpyroglutamate), menthylcarbonates (for example menthylpropyleneglycolcarbonate, menthylethyleneglycolcarbonate, menthylglycerolcarbonate or mixtures thereof), the semi-esters of menthols with a dicarboxylic acid or derivatives thereof (for example mono-menthylsuccinate, mono-menthylglutarate, mono-menthylmalonate, O-menthyl succinic acid ester-N,N-(dimethyl)amide, O-menthyl succinic acid ester amide), menthanecarboxylic acid amides (in this case preferably menthanecarboxylic acid-N-ethylamide [WS3] or N^{a}-(menthanecarbonyl)glycinethylester [WS5], as described in US 4,150,052, menthanecarboxylic acid-N-(4-cyanophenyl)amide or menthanecarboxylic acid-N-(4-cyanomethylphenyl)amide as described in WO 2005 049553 A1, methanecarboxylic acid-N-(alkoxyalkyl)amides), menthone and menthone derivatives (for example L-menthone glycerol ketal), 2,3-dimethyl-2-(2-propyl)-butyric acid derivatives (for example 2,3-dimethyl-2-(2-propyl)-butyric acid-N-methylamide [WS23]), isopulegol or its esters (l-(-)-isopulegol, l-(-)-isopulegolacetate), menthane derivatives (for example p-menthane-3,8-diol), cubebol or synthetic or natural mixtures, containing cubebol, pyrrolidone derivatives of cycloalkyldione derivatives (for example 3-methyl-2(1-pyrrolidinyl)-2-cyclopentene-1-one) or tetrahydropyrimidine-2-one (for example iciline or related compounds, as described in WO 2004/026840), further carboxamides (for example N-(2-(pyridin-2-yl)ethyl)-3-p-menthanecarboxamide or related compounds), (1R,2S,5R)-N-(4-Methoxyphenyl)-5-methyl-2-(1-isopropyl)-cyclohexane-carboxamide [WS12], oxamates (preferably those described in EP 2033688 A2).

### ANTI-INFLAMMATORY AGENTS

Suitable anti-inflammatory agents may be selected from the group formed by:
(i) Steroidal anti-inflammatory substances of the corticosteroid type, in particular hydrocortisone, hydrocortisone derivatives such as hydrocortisone 17-butyrate, dexamethasone, dexamethasone phosphate, methylprednisolone or cortisone,
(ii) non-steroidal anti-inflammatory substances, in particular oxicams such as piroxicam or tenoxicam, salicylates such as aspirin, disalcid, solprin or fendosal, acetic acid derivatives such as diclofenac, fenclofenac, indomethacin, sulindac, tolmetin or clindanac, fenamates such as mefenamic, meclofenamic, flufenamic or niflumic, propionic acid derivatives such as ibuprofen, naproxen or benoxaprofen, pyrazoles such as phenylbutazone, oxyphenylbutazone, febrazone or azapropazone,
(iii) natural or naturally occuring anti-inflammatory substances or substances that alleviate reddening and/or itching, in particular extracts or fractions from camomile, Aloe vera, Commiphora species, Rubia species, willow, willow-herb, oats, calendula, arnica, St John's wort, honeysuckle, rosemary, Passiflora incarnata, witch hazel, ginger or Echinacea, or single active compounds thereof,
(iv) histamine receptor antagonists, serine protease inhibitors (e.g. of Soy extracts), TRPV1 antagonists (e.g. 4-t-Butylcyclohexanol), NK1 antagonists (e.g. Aprepitant, Hydroxyphenyl Propamidobenzoic Acid), cannabinoid receptor agonists (e.g. Palmitoyl Ethanolamine) and TRPV3 antagonists.

### ANTI-MICROBIAL AGENTS

Suitable anti-microbial agents are, in principle, all substances effective against Gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxy-diphenyl ether (triclosan), 4-chloro-3,5-dimethyl-phenol, 2,2'-methylenebis(6-bromo-4-chlorophenol), 3-methyl-4-(1-methylethyl)phenol, 2-benzyl-4-chloro-phenol, 3-(4-chlorophenoxy)-1,2-propanediol, 3-iodo-2-propynyl butylcarbamate, chlorhexidine, 3,4,4'-trichlorocarbanilide (TTC), antibacterial fragrances, thymol, thyme oil, eugenol, oil of cloves, menthol, mint oil, farnesol, phenoxyethanol, glycerol monocaprate, glycerol monocaprylate, glycerol monolaurate (GML), diglycerol monocaprate (DMC), salicylic acid N-alkylamides, such as, for example, n-octylsalicylamide or n-decylsalicylamide.

### FILM FORMERS AND ANTI-DANDRUFF AGENTS

Standard film formers are, for example, chitosan, microcrystalline chitosan, quaternized chitosan, polyvinyl pyrrolidone, vinyl pyrrolidone/vinyl acetate copolymers, polymers of the acrylic acid series, quaternary cellulose derivatives, collagen, hyaluronic acid and salts thereof and similar compounds.

Suitable antidandruff agents are Pirocton Olamin (1-hydroxy-4-methyl-6-(2,4,4-trimethyl-pentyl)-2-(1H)-pyridinone monoethanolamine salt), Baypival^{®} (Climbazole), Ketoconazol^{®} (4-acetyl-1-{4-[2-(2,4-dichlorophenyl) r-2-(1H-imidazol-1-ylmethyl)-1,3-dioxylan-c-4-yl-methoxyphenyl)-piperazine, ketoconazole, elubiol, selenium disulfide, colloidal sulfur, sulfur polyethylene glycol sorbitan monooleate, sulfur ricinol polyethoxylate, sulfur tar distillate, salicylic acid (or in combination with hexachlorophene), undecylenic acid, monoethanolamide sulfosuccinate Na salt, Lamepon^{®} UD (protein/undecylenic acid condensate), zinc pyrithione, aluminium pyrithione and magnesium pyrithione/dipyrithione magnesium sulfate.

### CARRIERS AND HYDROTROPES

Preferred cosmetics carrier materials are solid or liquid at 25°C and 1013 mbar (including highly viscous substances) as for example glycerol, 1,2-propylene glycol, 1,2-butylene glycol, 1,3-propylene glycol, 1,3-butylene glycol, ethanol, water and mixtures of two or more of said liquid carrier materials with water. Optionally, the compositions described herein may be produced using preservatives or solubilizers. Other preferred liquid carrier substances, which may be a component of a composition as described herein are selected from the group consisting of oils such as vegetable oil, neutral oil and mineral oil.

Preferred solid carrier materials, which may be a component of a composition as described herein are hydrocolloids, such as starches, degraded starches, chemically or physically modified starches, dextrins, (powdery) maltodextrins (preferably with a dextrose equivalent value of 5 to 25, preferably of 10 - 20), lactose, silicon dioxide, glucose, modified celluloses, gum arabic, ghatti gum, traganth, karaya, carrageenan, pullulan, curdlan, xanthan gum, gellan gum, guar flour, carob bean flour, alginates, agar, pectin and inulin and mixtures of two or more of these solids, in particular maltodextrins (preferably with a dextrose equivalent value of 15 - 20), lactose, silicon dioxide and/or glucose.

In addition, hydrotropes, for example ethanol, isopropyl alcohol or polyols, may be used to improve flow behaviour. Suitable polyols preferably contain 2 to 15 carbon atoms and at least two hydroxyl groups. The polyols may contain other functional groups, more especially amino groups, or may be modified with nitrogen. Typical examples are
- glycerol;
- alkylene glycols such as, for example, ethylene glycol, diethylene glycol, propylene glycol, butylene glycol, hexylene glycol and polyethylene glycols with an average molecular weight of 100 to 1000 Dalton;
- technical oligoglycerol mixtures with a degree of self-condensation of 1.5 to 10, such as for example technical diglycerol mixtures with a diglycerol content of 40 to 50% by weight;
- methylol compounds such as, in particular, trimethylol ethane, trimethylol propane, trimethylol butane, pentaerythritol and dipentaerythritol;
- lower alkyl glucosides, particularly those containing 1 to 8 carbon atoms in the alkyl group, for example methyl and butyl glucoside;
- sugar alcohols containing 5 to 12 carbon atoms, for example sorbitol or mannitol,
- sugars containing 5 to 12 carbon atoms, for example glucose or sucrose;
- amino sugars, for example glucamine;
- dialcoholamines, such as diethanolamine or 2-aminopropane-1,3-diol.

### PRESERVATIVES

Suitable preservatives are, for example, phenoxyethanol, formaldehyde solution, parabens, (additional) pentanediol or sorbic acid and the other classes of compounds listed in Appendix 6, Parts A and B of the Kosmetikverordnung ("Cosmetics Directive").

### PERFUME OILS AND FRAGRANCES

The compositions as described herein may also contain suitable perfume oils, i.e. mixtures of natural and/or synthetic perfumes. Natural perfumes include the extracts of blossoms (lily, lavender, rose, jasmine, neroli, ylang-ylang), stems and leaves (geranium, patchouli, petitgrain), fruits (anise, coriander, caraway, juniper), fruit peel (bergamot, lemon, orange), roots (nutmeg, angelica, celery, cardamom, costus, iris, calmus), woods (pinewood, sandalwood, guaiac wood, cedarwood, rosewood), herbs and grasses (tarragon, lemon grass, sage, thyme), needles and branches (spruce, fir, pine, dwarf pine), resins and balsams (galbanum, elemi, benzoin, myrrh, olibanum, opoponax). Animal raw materials, for example civet and beaver, may also be used. Typical synthetic perfume compounds are products of the ester, ether, aldehyde, ketone, alcohol and hydrocarbon type. Examples of perfume compounds of the ester type are benzyl acetate, phenoxyethyl isobutyrate, p-tert.butyl cyclohexylacetate, linalyl acetate, dimethyl benzyl carbinyl acetate, phenyl ethyl acetate, linalyl benzoate, benzyl formate, ethylmethyl phenyl glycinate, allyl cyclohexyl propionate, styrallyl propionate and benzyl salicylate. Ethers include, for example, benzyl ethyl ether while aldehydes include, for example, the linear alkanals containing 8 to 18 carbon atoms, citral, citronellal, citronellyloxyacetaldehyde, cyclamen aldehyde, hydroxycitronellal, lilial and bourgeonal. Examples of suitable ketones are the ionones, α-isomethylionone and methyl cedryl ketone. Suitable alcohols are anethol, citronellol, eugenol, isoeugenol, geraniol, linalool, phenylethyl alcohol and terpineol. The hydrocarbons mainly include the terpenes and balsams. However, it is preferred to use mixtures of different perfume compounds which, together, produce an agreeable perfume. Other suitable perfume oils are essential oils of relatively low volatility which are mostly used as aroma components. Examples are sage oil, camomile oil, clove oil, melissa oil, mint oil, cinnamon leaf oil, lime-blossom oil, juniper berry oil, vetiver oil, olibanum oil, galbanum oil, ladanum oil and lavendin oil. The following are preferably used either individually or in the form of mixtures: bergamot oil, dihydromyrcenol, lilial, lyral, citronellol, phenylethyl alcohol, hexylcinnamaldehyde, geraniol, benzyl acetone, cyclamen aldehyde, linalool, Boisambrene Forte, Ambroxan, indole, hedione, sandelice, citrus oil, mandarin oil, orange oil, allylamyl glycolate, cyclovertal, lavendin oil, clary oil, damascone, geranium oil bourbon, cyclohexyl salicylate, Vertofix Coeur, Iso-E-Super, Fixolide NP, evernyl, iraldein gamma, phenylacetic acid, geranyl acetate, benzyl acetate, rose oxide, romillat, irotyl and floramat.

### DYES

Suitable dyes that may be contained in the compositions as described herein are any of the substances suitable and approved for cosmetic purposes as listed, for example, in the publication "Kosmetische Färbemittel" of the Farbstofflcommission der Deutschen Forschungsgemeinschaft, Verlag Chemie, Weinheim, 1984, pages 81 to 106. Examples include cochineal red A (C.I. 16255), patent blue V (C.I. 42051), indigotin (C.I. 73015), chlorophyllin (C.I. 75810), quinoline yellow (C.I. 47005), titanium dioxide (C.I. 77891), indanthrene blue RS (C.I. 69800) and madder lake (C.I. 58000). Luminol may also be present as a luminescent dye. Advantageous colored pigments are for example titanium dioxide, mica, iron oxides (e.g. Fe₂O₃ Fe₃O₄, FeO(OH)) and/or tin oxide. Advantageous dyes are for example carmine, Berlin blue, chromium oxide green, ultramarine blue and/or manganese violet.

The auxiliary substances and additives described above may be included in the compositions as described herein in quantities of up to 99 wt.-%, preferably 5 to 99 wt.-%, more preferably 10 to 80 wt.-%, based on the total weight of the composition. The amounts of cosmetic or dermatological auxiliary agents and additives and perfume to be used in each case can easily be determined by the person skilled in the art by simple trial and error, depending on the nature of the particular product.

The compositions as described herein can also contain water in a quantity of up to 99 wt.-%, preferably 5 to 80 wt.-%, based on the total weight of the composition.

Another preferred embodiment according to the invention is a use as described herein, wherein the extract is obtained or obtainable by a method consisting of or comprising the steps of extracting cell material of Isochrysis sp., preferably Tahitian Isochrysis, with a liquid extractant,
wherein the extraction comprises
   a) exposition of the cell material to the extractant for up to 24h at a temperature of 50°C or less, and
   b) removal of the cell material to obtain an extract, the extract being the composition or being further processed into the composition,
and wherein preferably the cell material of Isochrysis sp. and/or Tahitian Isochrysis is obtainable or obtained by a method consisting of or comprising the steps:
   i) Cultivating Isochrysis sp. cells and/or preferably Tahitian Isochrysis cells,
   ii) harvesting the cells to obtain completely or substantially intact cell material,
   iii) optionally washing the cell material of step ii) once or multiple times, to obtain washed, substantially or completely intact cell material,
   iv) optionally freeze-drying the cell material of step ii) and/or step iii), if present.

Another preferred embodiment according to the invention is a use as described herein, wherein the liquid extractant is selected from the group consisting of hexane, ethyl acetate, water, methanol, and mixtures of two or more of these extractants.

Another preferred embodiment according to the invention is a use as described herein, wherein the extraction comprises repeating steps a) and b) once, twice, three or four times, and wherein in each step a) the cell material removed in the respective prior step b) is used, and the extracts of steps b) are combined.

Another preferred embodiment according to the invention is a use as described herein, wherein for extraction cell material is obtained in step b) of a prior extraction with a different extractant.

Another preferred embodiment according to the invention is a use as described herein, wherein the cell material used in the first step a) is freeze-dried Isochrysis sp., preferably Tahitian Isochrysis.

Another preferred embodiment according to the invention is a use as described herein, wherein the composition comprises 0.00001 to 10 wt.-%, preferably 0.0005 to 1 wt.-% and most preferably 0.001 to 0.01 wt.-% extract, preferably dry extract, based on the total weight of the composition.

Another preferred embodiment according to the invention is a use as described herein, wherein the composition also comprises 1,2-pentanediol, preferably in an amount in the range of from 0.001 to 99.9 wt.-%, more preferably 10 to 99.9 wt.-%, more preferably 25 to 99.8 wt.-%, and most preferably 50 to 99.7 wt.-%, based on the total weight of the composition.

The present invention also relates to a method for influencing or modifying the hair fiber of human hair consisting of or comprising the following steps:
a) Providing a composition consisting of or comprising an extract of Isochrysis sp., preferably of Tahitian Isochrysis,
   preferably wherein the composition is a hair care product, more preferably is a shampoo (including anti-dandruff shampoo, baby shampoo, shampoo for dry scalps, concentrated shampoo, dry formulation of a shampoo), conditioner, preferably a leave-on or leave-in conditioner (or a dry formulation of a conditioner or derivative thereof), 2-in-1 product (comprising shampoo and conditioner), tip repairing product, hair treatment cure, hair tonic, hair lotion, hair rinse, styling cream, hair setting composition, preferably formulated as gel, mousse or spray, styling aid, blonding composition or hair coloring composition, most preferably is a shampoo, conditioner, preferably a leave-on or leave-in conditioner, or a 2-in-1 product (comprising shampoo and conditioner), and/or
   preferably wherein the composition comprises 0.00001 to 10 wt.-%, preferably 0.0005 to 1 wt.-% and most preferably 0.001 to 0.01 wt.-% extract, preferably dry extract, based on the total weight of the composition, and/or preferably wherein the composition also comprises 1,2-pentanediol, preferably in an amount in the range of from 0.001 to 99.9 wt.-%, more preferably 10 to 99.9 wt.-%, more preferably 25 to 99.8 wt.-%, and most preferably 50 to 99.7 wt.-%, based on the total weight of the composition,
b) applying the composition of step a) to the human hair,
c) optionally rinsing the human hair to remove parts or all of the composition from the human hair,
d) optionally repeating steps a) to c), if present, 1, 2, 3, 4, 5 or more times,
e) optionally drying the human hair,
wherein the influencing or modifying the hair fiber of human hair is defined as set forth in appended claim 11.

Another preferred embodiment according to the invention is a method as described herein, alternative (v), wherein the physical stress originates or originated from combing, drying, heating, curling or straightening the hair fibers, or the use of retaining means such as hair clips, hair curlers or hair ties and/or the chemical stress originates or originated from hair care products such as shampoos, conditioners, hair treatment cures, hair tonics, hair lotions, hair rinses, styling creams, hair setting compositions, styling aids, blonding compositions or hair colouring compositions and/or the environmental stress originates or originated from exposure to sun, industrial pollution, sea water or chlorinated water.

Another preferred embodiment according to the invention is a method as described herein, wherein the extract in step a) is obtained or obtainable by a method as defined above.

Preferred embodiments of the use according to the invention correspond to or can be derived from the preferred embodiments of the method according to the invention, which are explained above, or *vice versa.*

The invention will now be described in more detail hereinafter with references to the examples.

### EXAMPLES

### Formulation examples

### 1) Shampoo (amounts in wt.-%)

| **Ingredients** | **Amount** |
|---|---|
| Sodium lauryl ether sulfate (e.g. Texapon NSO) | 12 |
| Cocamidopropyl betaine (e.g. Dehyton K) | 2 |
| Sodium chloride | 1.4 |
| Citric acid | 1.3 |
| Perfume oil | 0.3 |
| Phenoxyethanol. methyl-. ethyl-. butyl- and propylparaben | 0.5 |
| Isochrysis galbana extract in 1,2-pentanediol (dry extract content 0.5 wt.%) | 1.0 |
| Water | Ad 100 |

### 2) 2-in-1-Shampoo (amounts in wt.-%)

| **Ingredients** | **INCI Name** | **Amount** |
|---|---|---|
| Deionized water | Water | Ad 100 |
| Plantacare PS 10 | Sodium Laureth Sulfate. Lauryl Glucoside | 20.0 |
| Euperlan PK 771 | Glycol Distearate. Sodium Lauryl Sulfate. Cocamide MEA. Laureth-10 | 6.0 |
| Sodium chloride | Sodium Chloride | 1.4 |
| Citric acid monohydrate crystalline | Citric acid | 0.1 |
| Perfume oil | Fragrance | 0.5 |
| Dragocid Liquid | Phenoxyethanol, Parabens | 0.5 |
| Isochrysis galbana extract in 1,2-pentanediol (dry extract content 0.5 wt.%) | - | 2.0 |

### 3) Anti-dandruff Shampoo (amounts in wt.-%)

| **Ingredients** | **Amount** |
|---|---|
| Climbazole | 0.50 |
| Sodium Laureth Sulfate | 37.00 |
| Cocamidopropyl Betaine | 8.00 |
| PEG-6 Caprylic/Capric Glycerides | 2.50 |
| Laureth-2 | 2.00 |
| Water (Aqua). Glycerol. Thymus Vulgaris (Thyme). Flower/Leaf Extract | 0.50 |
| Rosmarinus Officinalis (Rosemary) Leaf Water. Water (Aqua). Butylene Glycol. Pentylene Glycol | 0.50 |
| Bisabolol | 0.10 |
| Panthenol | 0.50 |
| Polyquaternium-10 | 0.40 |
| Perfume oil | 0.50 |
| Phenoxyethanol. Methylparaben. Ethylparaben. Butylparaben. Propylparaben. Isobutylparaben | 0.70 |
| Isochrysis galbana extract in 1 ,2-pentanediol (dry extract content 0.5 wt.%) | 1.00 |
| Water (Aqua) | Ad 100 |

### 4) Hair conditioner with Crinipan, rinse-off (amounts in wt.-%)

| **Ingredients** | **INCl Name** | **Amount** |
|---|---|---|
| Lanette^{®} O | Cetearyl Alcohol | 4.00 |
| Dragoxat 89 | Ethylhexyl Isononanoate | 2.00 |
| Genamin^{®} KDM-P | Behentrimonium Chloride | 1.00 |
| SF 1550 | Phenyl Trimethicone | 0.10 |
| Neo Heliopan^{®} BB | Benzophenone-3 | 0.10 |
| Crinipan^{®} AD | Climbazole | 0.80 |
| Glycerol 99.5 P. | Glycerol | 6.00 |
| Water | Water (Aqua) | Ad 100 |
| Actipone^{®} Alpha Pulp | Water (Aqua). Butylene Glycol. Malic Acid. Actinidia Chinensis (Kiwi)Fruit Juice. Citrus. Aurantium Dulcis (Orange). Juice. Citrus Paradisi (Grapefruit) Juice. Pyrus Malus (Apple) Juice. Trideceth-9. PrunusAmygdalus Dulcis (Sweet Almond) Seed Extract | 0.50 |
| Extrapone^{®} Bamboo P | Propylene Glycol. Water (Aqua). Butylene Glycol. Bambusa Vulgaris Shoot Extract | 0.50 |
| Sodium Hydroxide 10% solution | Sodium Hydroxide | 0.40 |
| Colour I | Colour | 0.60 |
| Colour II | Colour | 0.30 |
| Perfume oil | Fragrance | 0.40 |
| Preservative | Methylparaben | 0.20 |
| Isochrysis galbana extract in 1,2-pentanediol (dry extract content 0.5 wt.%) | | 1.00 |

### 5) Sprayable hair conditioner with zinc pyrithrione, leave-on (amounts in wt.-%)

| **Ingredients** | **INCl Name** | **Amount** |
|---|---|---|
| Monomuls 60-35 C | Hydrogenated Palm Glycerides | 1.70 |
| Cetiol OE | Dicaprylyl Ether | 7.20 |
| Abil 100 | Dimethicone | 3.60 |
| Dehyquart F 75 | Distearoylethyl Hydroxyethylmonium. | 4.00 |
| | Methosulfate. Cetearyl Alcohol | |
| Eumulgin B1 | Ceteareth-12 | 3.50 |
| Cetiol S | Diethylhexylcyclohe xane | 7.20 |
| D-Panthenol | Panthenol | 0.10 |
| Glycerol 99.5 P. | Glycerol | 1.50 |
| Water | Water (Aqua) | Ad 100 |
| Actipone^{®} Rosemary | Water (Aqua). Propylene. Glycol. | 0.10 |
| | Rosmarinus Officinalis. (Rosemary) Leaf Extract | |
| Frescolat^{®} ML Cryst. | Menthyl Lactate | 0.50 |
| Dragosantol100 | Bisabolol | 0.10 |
| Zinc Omadine | Zinc pyrithione | 0.10 |
| Perfume oil | Fragrance | 0.40 |
| Phenonip^{®} | phenoxyethanol. methylparaben. | 0.30 |
| | ethylparaben. butylparaben. | |
| | propylparaben. isobutylparaben | |
| Isochrysis galbana extract in 1,2-pentanediol (dry extract content 0.5 wt.%) | 1,2-pentanediol, Isochrysis galbana extract | 1.00 |

### 6) Hair conditioner with UV protection (amounts in wt.-%)

| **Ingredients** | **INCl Name** | **Amount** |
|---|---|---|
| Renex PEG 6000 | PEG-150 | 2.50 |
| Hair Conditioner Base | Cetyl alcohol. behentrimonium chloride. Triticum Vulgare (Wheat) bran extract. linoleic acid | 3.00 |
| PCL-Solid | Stearyl heptanoate. stearyl caprylate | 0.50 |
| Dow Corning 5200 | Laurylmethicone copolyol | 0.50 |
| Natrosol 250 HR | Hydroxyethylcellulose | 0.50 |
| Benzophenone-4 | Benzophenone-4 | 1.00 |
| Neo Heliopan AP | Disodiumphenyldibenz-imidazole tetrasulphonate | 1.00 |
| Amino methyl propanol | Amino methyl propanol | 2.00 |
| Dow Corning 949 cationic emulsion | Amodimethicone. cetrimonium chloride. trideceth-12 | 2.00 |
| Perfume oil | Fragrance | 0.80 |
| 1.2-hexanediol | 1.2-hexanediol | 0.50 |
| Isochrysis galbana extract in 1 ,2-pentanediol (dry extract content 0.5 wt.%) | | 1.00 |
| Water | Water (Aqua) | Ad 100 |

### Test results

### Combability test

The hair combability test measures the force required for a comb to pass through a hair tress.

The parameter measured is:
- Total work: the total energy required during the whole combing process (Joules)

Since the hair surface characteristics are dependent on the cuticle/comb interaction, hair in wet or dry situations can change values in the measurements.

Five Caucasian bleached hair tresses (5 g, 20 cm) were cleaned with Sodium Lauryl Ether Sulfate (SLES) 10% solution for 1 minute. Then exemplary formulations of the present invention were applied five times in each tress (0.5 mL of formulation + massaging for 1 minute, followed by 1 minute of rinsing at running water at 30 °C, leave-on conditioner was not rinsed), respectively. Test in wet condition was performed. Tresses were then cleaned and test products were applied again in order to avoid variation in the amount of test product for the dry condition test. Tresses were left at controlled environment (22 °C, ± 2 °C; 50% relative humidity (RH), ± 5%) overnight for drying. Test in dry condition was performed. The energy involved in the combing process was extracted from each measurement. Comparisons among the tests were performed, using analysis of variance, with post test of Tukey, considering 95% confidence interval.

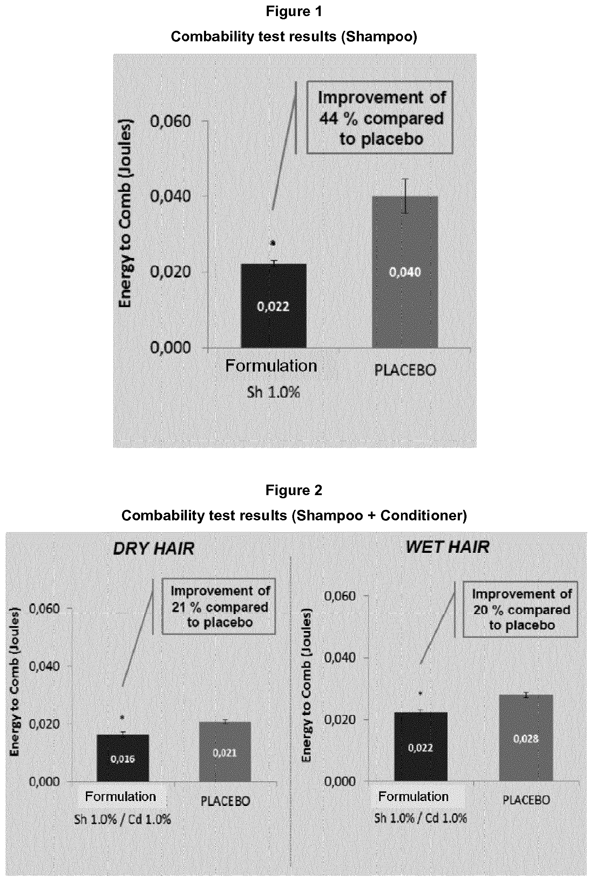

**Figure 1** shows the test results for an exemplary shampoo formulation (comprising 1.0 wt.-% of extract as defined in the above formulations) used according to the invention. As can be taken from the graph, the improvement in combability compared to placebo in dry hair was 44%.

**Figure 2** shows the test results for an exemplary 2-in-1 product (shampoo + conditioner, comprising 2.0 wt.-% of extract as defined in the above formulations) used according to the invention. As can be taken from the graph, the improvement in combability compared to placebo in dry hair was 21% (left). The improvement in combability compared to placebo in wet hair was 20% (right).

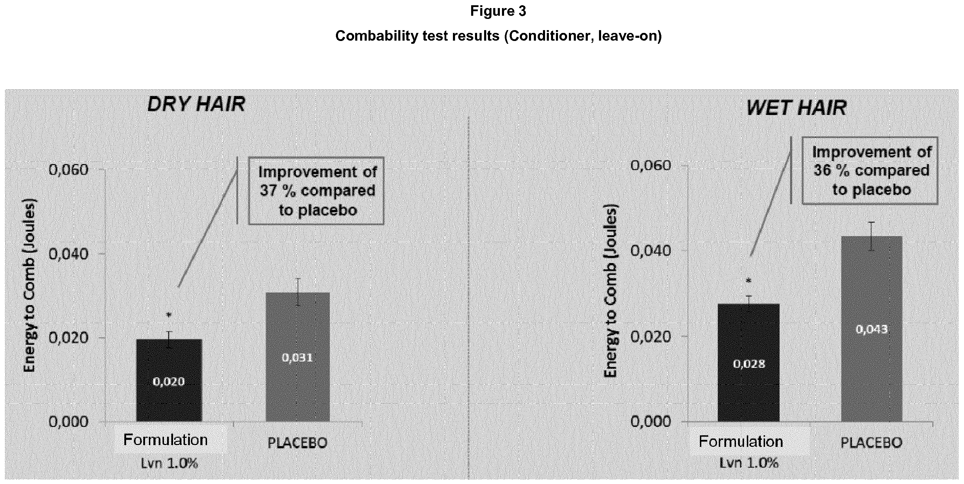

**Figure 3** shows the test results for an exemplary conditioner (leave-on, comprising 1.0 wt.-% of extract as defined in the above formulations) used according to the invention. As can be taken from the graph, the improvement in combability compared to placebo in dry hair was 37% (left). The improvement in combability compared to placebo in wet hair was 36% (right).

### Volume/frizz control assessment

Volume is the gain of body in a hairtress due to its humidity exposure or mechanical actions such as combing or friction of fibers. Frizz is the bristling of some fibers of hair that get apart from the tress body (fly-away effect). The variation in the shape of the hair tress is given by the variation in the number of pixels in the images taken before and after the natural drying.

The parameter measured is:
- Number of pixels in the image, as expression of the gain of volume.

Five afro hair tresses (5 g, 15 cm) were cleaned with SLES 10% solution for 1 minute. Then exemplary formulations of the present invention were applied five times in each tress (0.5 mL of formulation + massaging for 1 minute, followed by 1 minute of rinsing at running water at 30 °C, leave-on conditioner was not rinsed), respectively. Images at initial time (T0) were captured by Rumba System and Digital Camera Nikon D5200. The tresses were then left at controlled environment (22 °C, ± 2 °C; 50% RH, ± 5%) overnight for drying. Images at final time (T1) were then captured in both mentioned systems. The difference in the number of pixels between T1 and T0 was calculated for each tress. Comparisons among the tests were performed, using analysis of variance, with post test of Tukey, considering 95% confidence interval.

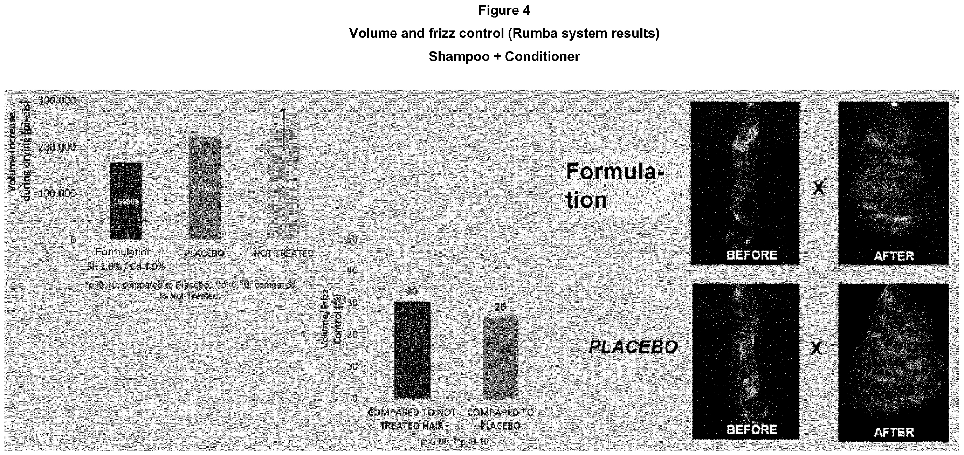

**Figure 4** shows that the volume increase (in pixels) of the tested hair tresses during drying was significantly lower after use of an exemplary 2-in-1 product (shampoo + conditioner, comprising 2.0 wt.-% of extract as defined in the above formulations) according to the present invention compared to both placebo and non-treated hair tresses. Also the volume/frizz control (in %) compared to non-treated hair and placebo was significantly higher (left). The right side of the image shows images of the treated and placebo hair tresses captured with the Rumba system before and after drying.

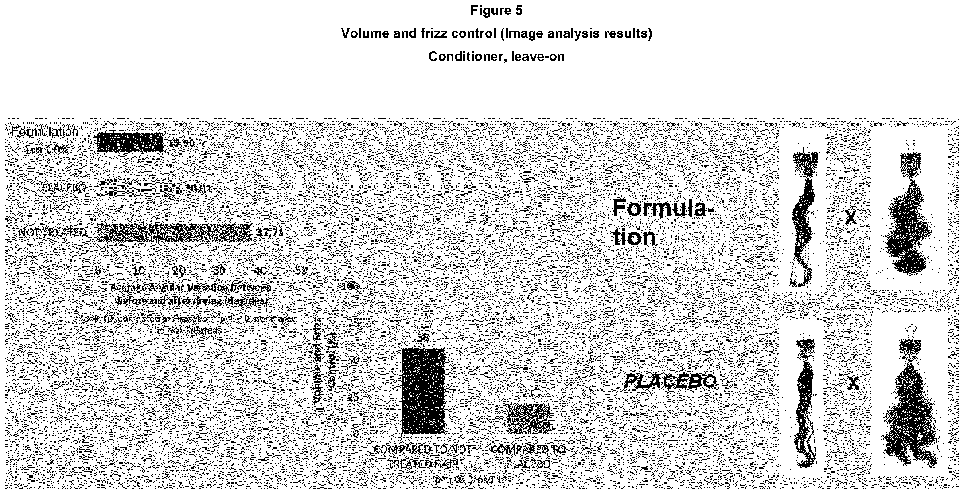

**Figure 5** shows that the average angular variation (in degrees) between before and after drying of the hair tresses was significantly lower after use of an exemplary conditioner (leave-on, comprising 1.0 wt.-% of extract as defined in the above formulations) according to the present invention compared to both placebo and non-treated hair tresses. Also the volume/frizz control (in %) compared to non-treated hair and placebo was significantly higher (left). The right side of the image shows digital camera images of the treated and placebo hair tresses before and after drying.

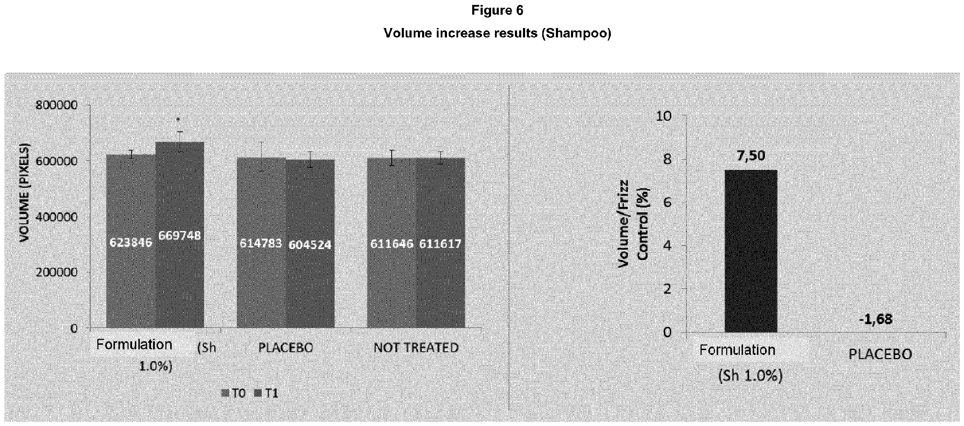

**Figure 6** shows that after use of an exemplary shampoo formulation (comprising 1.0 wt.-% of extract as defined in the above formulations) according to the present invention the volume (in pixels) of the treated hair tresses increased at a statistically significant extent (left) and the volume/frizz control (in %) also increased (right).

### Tensile test

The tensile test refers to a method which measures different linear mechanical properties of the hair fiber.

The parameters measured were:
- Elastic Module (N/m²): measurement of the stiffness of the hair fiber.
- Stress at 15% Strain (gmf/µm²): measurement of the stress when the fiber has been strained 15%.
- Break Extension (%): the maximun extension before break.
- Break Stress (gmf/µm²): the maximum stress the hair fiber is able to resist, measured at the breaking point.

Three Caucasian bleached hair tresses (5 g, 20 cm) were cleaned with SLES 10% solution for 1 minute. Then exemplary formulations of the present invention were applied five times in each tress (0.5 mL of formulation + massaging for 1 minute, followed by 1 minute of rinsing at running water at 30 °C, leave-on conditioner was not rinsed), respectively. The tresses were left at controlled environment (22°C, ± 2°C; 50% RH, ± 5%) overnight for drying. 45 fibers from each treatment were collected randomly. They were fixed in metallic crimps one by one. Diameter measurements were executed and then the tensile test was performed. Comparisons among the tests were performed, using analysis of variance, with post test of Tukey, considering 95% confidence interval.

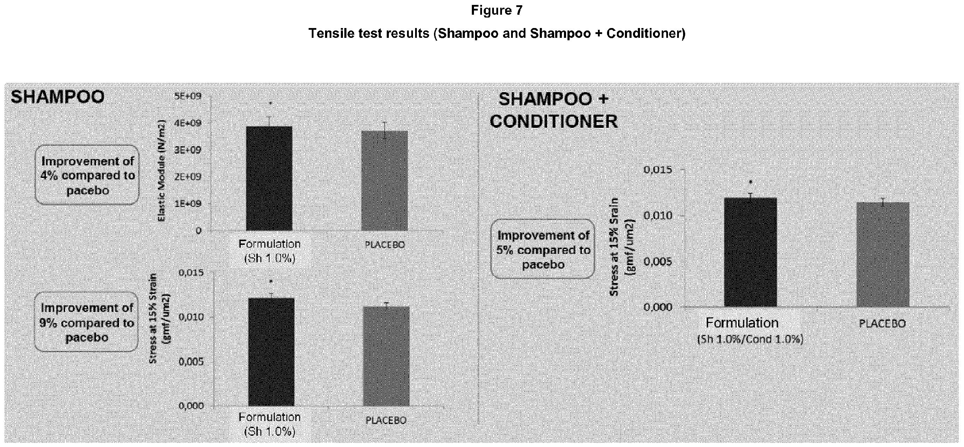

**Figure 7** shows that after use of an exemplary shampoo formulation (comprising 1.0 wt.-% of extract as defined in the above formulations) according to the present invention the elastic module (in N/m²) of the hair fibers improved by 4% compared to placebo and the stress at 15% strain (in gmf/µm²) improved by 9% compared to placebo (left). After use of an exemplary 2-in-1 product (shampoo + conditioner, comprising 2.0 wt.-% of extract as defined in the above formulations) the stress at 15% strain (in gmf/µm²) improved by 5% compared to placebo (right).

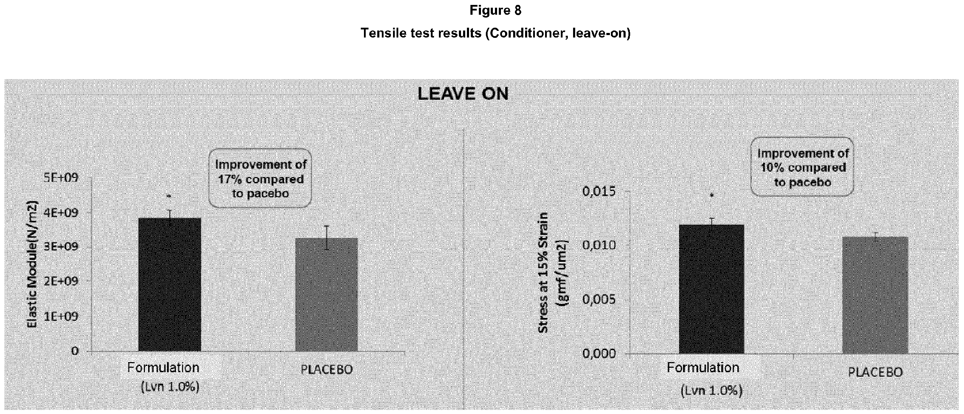

**Figure 8** shows that after use of an exemplary conditioner (leave-on, comprising 1.0 wt.-% of extract as defined in the above formulations) according to the present invention the elastic module (in N/m²) of the hair fibers improved by 17% compared to placebo (left) and the stress at 15% strain (in gmf/µm²) improved by 10% compared to placebo (right).

### Breakage test

The breakage test subjects the hair to 25000 cycles of combing in order to provoke mechanical stress that will lead to fiber breakage. The broken fragments are collected and quantified. The number of broken fragments is an expression of the hair fiber weakness.

Five Caucasian bleached hair tresses (5 g, 20 cm) were cleaned with SLES 10% solution for 1 minute. Then exemplary formulations of the present invention were applied five times in each tress (0.5 mL of formulation + massaging for 1 minute, followed by 1 minute of rinsing at running water at 30 °C, leave-on conditioner was not rinsed). The tresses were left at controlled environment (22°C, ± 2°C; 50% RH, ± 5%) overnight for drying. The tresses were subsequently submitted to five cycles of 5000 combings in the combing machine using brushes. After each cycle, the machine was stopped and the fragments of hair were collected and counted manually. Comparisons among the tests were performed, using analysis of variance, with post test of Tukey, considering 95% confidence interval.

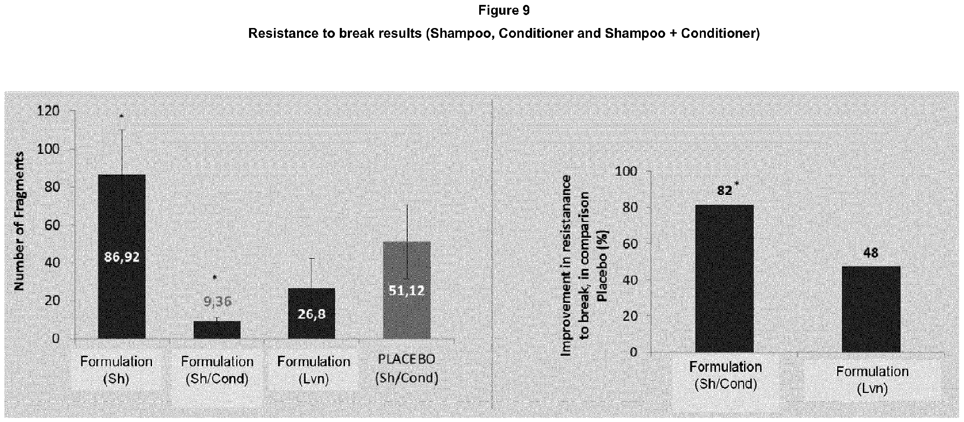

**Figure 9** shows the number of fragments collected after the breakage test of hair tresses that have been treated with an exemplary shampoo formulation (comprising 1.0 wt.-% of extract as defined in the above formulations), or an exemplary 2-in-1 product (shampoo + conditioner, comprising 2.0 wt.-% of extract as defined in the above formulations), or an exemplary conditioner (leave-on, comprising 1.0 wt.-% of extract as defined in the above formulations) according to the present invention (left). The improvement in resistance to breakage in comparison to placebo was particularly pronounced after the use of an exemplary 2-in-1 product (shampoo + conditioner, comprising 2.0 wt.-% of extract as defined in the above formulations) and after the use of an exemplary conditioner (leave-on, comprising 1.0 wt.-% of extract as defined in the above formulations) according to the present invention (right).

## Claims

1. Use of a composition consisting of or comprising an extract of Isochrysis sp., preferably Tahitian Isochrysis, for influencing or modifying the hair fiber of human hair, wherein the use is not a use for influencing or modifying growth of human hair or pigmentation of human hair, wherein the composition comprises 0.00001 to 10 wt.-% extract, preferably dry extract, based on the total weight of the composition, and
wherein the composition is used to
(i) improve the combability of human hair and/or
(ii) control the volume of human hair fibers and/or
(iii) avoid or reduce the frizz of human hair and/or
(iv) improve the tensile strength of human hair fibers and/or
(v) decrease the fiber breakage of human hair, preferably during or after exposure of the hair fiber to physical and/or chemical and/or environmental stress.

2. Use according to claim 1, alternative (v), wherein the physical stress originates or originated from combing, drying, heating, curling or straightening the hair fibers, or the use of retaining means such as hair clips, hair curlers or hair ties and/or the chemical stress originates or originated from hair care products such as shampoos, conditioners, hair treatment cures, hair tonics, hair lotions, hair rinses, styling creams, hair setting compositions, styling aids, blonding compositions or hair colouring compositions and/or the environmental stress originates or originated from exposure to sun, industrial pollution, sea water or chlorinated water.

3. Use according to any of the preceding claims, wherein the composition is a hair care product, more preferably is a shampoo, conditioner, preferably a leave-on or leave-in conditioner, 2-in-1 product, tip repairing product, hair treatment cure, hair tonic, hair lotion, hair rinse, styling cream, hair setting composition, preferably formulated as gel, mousse or spray, styling aid, blonding composition or hair coloring composition, most preferably is a shampoo, conditioner, preferably a leave-on or leave-in conditioner, or a 2-in-1 product.

4. Use according to any of the preceding claims, wherein the extract is obtained or obtainable by a method consisting of or comprising the steps of extracting cell material of Isochrysis sp., preferably Tahitian Isochrysis, with a liquid extractant,
wherein the extraction comprises
a) exposition of the cell material to the extractant for up to 24h at a temperature of 50°C or less, and
b) removal of the cell material to obtain an extract, the extract being the composition or being further processed into the composition,
and wherein preferably the cell material of Isochrysis sp. and/or Tahitian Isochrysis is obtainable or obtained by a method consisting of or comprising the steps:
i) Cultivating Isochrysis sp. cells and/or preferably Tahitian Isochrysis cells,
ii) harvesting the cells to obtain completely or substantially intact cell material,
iii) optionally washing the cell material of step ii) once or multiple times, to obtain washed, substantially or completely intact cell material,
iv) optionally freeze-drying the cell material of step ii) and/or step iii), if present.

5. Use according to claim 4, wherein the liquid extractant is selected from the group consisting of hexane, ethyl acetate, water, methanol, and mixtures of two or more of these extractants.

6. Use according to claim 4 or 5, wherein the extraction comprises repeating steps a) and b) once, twice, three or four times, and wherein in each step a) the cell material removed in the respective prior step b) is used, and the extracts of steps b) are combined.

7. Use according to any of the claims 4 to 6, wherein for extraction cell material is obtained in step b) of a prior extraction with a different extractant.

8. Use according to any of the claims 4 to 7, wherein the cell material used in the first step a) is freeze-dried Isochrysis sp., preferably Tahitian Isochrysis.

9. Use according to any of the preceding claims, wherein the composition comprises 0.0005 to 1 wt.-% and most preferably 0.001 to 0.01 wt.-% extract, preferably dry extract, based on the total weight of the composition.

10. Use according to any of the preceding claims, wherein the composition also comprises 1,2-pentanediol, preferably in an amount in the range of from 0.001 to 99.9 wt.-%, more preferably 10 to 99.9 wt.-%, more preferably 25 to 99.8 wt.-%, and most preferably 50 to 99.7 wt.-%, based on the total weight of the composition.

11. Method for influencing or modifying the hair fiber of human hair consisting of or comprising the following steps:
a) Providing a composition consisting of or comprising an extract of Isochrysis sp., preferably of Tahitian Isochrysis,
preferably wherein the composition is a hair care product, more preferably is a shampoo, conditioner, preferably a leave-on or leave-in conditioner, 2-in-1 product, tip repairing product, hair treatment cure, hair tonic, hair lotion, hair rinse, styling cream, hair setting composition, preferably formulated as gel, mousse or spray, styling aid, blonding composition or hair coloring composition, most preferably is a shampoo, conditioner, preferably a leave-on or leave-in conditioner, or a 2-in-1 product, and/or
preferably wherein the composition comprises 0.00001 to 10 wt.-%, preferably 0.0005 to 1 wt.-% and most preferably 0.001 to 0.01 wt.-% extract, preferably dry extract, based on the total weight of the composition, and/or preferably wherein the composition also comprises 1,2-pentanediol, preferably in an amount in the range of from 0.001 to 99.9 wt.-%, more preferably 10 to 99.9 wt.-%, more preferably 25 to 99.8 wt.-%, and most preferably 50 to 99.7 wt.-%, based on the total weight of the composition,
b) applying the composition of step a) to the human hair,
c) optionally rinsing the human hair to remove parts or all of the composition from the human hair,
d) optionally repeating steps a) to c), if present, 1, 2, 3, 4, 5 or more times,
e) optionally drying the human hair,
wherein the influencing or modifying the hair fiber of human hair is:
(i) an improvement of the combability of human hair and/or
(ii) a control of the volume of human hair fiber and/or
(iii) an avoidance or reduction the frizz of human hair and/or
(iv) an improvement of the tensile strength of human hair fiber and/or
(v) a decrease of the fiber breakage of human hair, preferably during or after exposure of the hair fibers to physical and/or chemical and/or environmental stress.

12. Method according to claim 11, alternative (v), wherein the physical stress originates or originated from combing, drying, heating, curling or straightening the hair fibers, or the use of retaining means such as hair clips, hair curlers or hair ties and/or the chemical stress originates or originated from hair care products such as shampoos, conditioners, hair treatment cures, hair tonics, hair lotions, hair rinses, styling creams, hair setting compositions, styling aids, blonding compositions or hair colouring compositions and/or the environmental stress originates or originated from exposure to sun, industrial pollution, sea water or chlorinated water.

13. Method according to any of the claims 11 to 12, wherein the extract in step a) is obtained or obtainable by a method as defined in any one of claims 4 to 8.

## Patentansprüche

1. Verwendung einer Zusammensetzung, die aus einem Extrakt von Isochrysis sp., vorzugsweise Tahiti-Isochrysis, besteht oder diesen umfasst, zur Beeinflussung oder Modifizierung der Haarfaser von menschlichem Haar, wobei die Verwendung keine Verwendung zur Beeinflussung oder Modifizierung des Wachstums von menschlichem Haar oder der Pigmentierung von menschlichem Haar ist, wobei die Zusammensetzung 0,00001 bis 10 Gew.-% Extrakt, vorzugsweise Trockenextrakt, bezogen auf das Gesamtgewicht der Zusammensetzung, enthält, und wobei die Zusammensetzung verwendet wird zur
(i) Verbesserung der Kämmbarkeit von menschlichem Haar und/oder
(ii) Kontrolle des Volumens der menschlichen Haarfasern und/oder
(iii) Vermeidung oder Reduzierung des Kräuseln von menschlichem Haar und/oder
(iv) Verbesserung der Zugfestigkeit von menschlichen Haarfasern und/oder
(v) Verringerung des Faserbruchs von menschlichem Haar, vorzugsweise während oder nach der Exposition der Haarfaser gegenüber physikalischen und/oder chemischen und/oder umweltbedingten Belastungen.

2. Verwendung nach Anspruch 1, Alternative (v), wobei die physikalische Belastung durch Kämmen, Trocknen, Erhitzen, Lockenwickeln oder Glätten der Haarfasern oder durch die Verwendung von Haltemitteln wie Haarspangen, Lockenwicklern oder Haargummis entsteht oder entstanden ist und/oder die chemische Belastung durch Haarpflegemittel wie Shampoos, Spülungen, Haarkuren, Haarwasser, Haarlotionen, Haarspülungen, Stylingcremes, Haarfestiger, Stylinghilfe, Blondiermittel oder Haarfärbemittel entsteht oder entstanden ist und/oder die Umweltbelastung durch Sonneneinstrahlung, industrielle Verschmutzung, Meerwasser oder chlorhaltiges Wasser entsteht oder entstanden ist.

3. Verwendung nach einem der vorangehenden Ansprüche, wobei die Zusammensetzung ein Haarpflegemittel, bevorzugter ein Shampoo, eine Spülung, vorzugsweise eine Leave-on oder Leave-in Spülung, ein 2-in-1-Produkt, ein Spitzenreparaturmittel, eine Haarkur, ein Haartonikum, eine Haarlotion, eine Haarspülung, eine Stylingcreme, ein Haarfestiger, vorzugsweise als Gel, Mousse oder Spray formuliert, eine Stylinghilfe, ein Blondiermittel oder ein Haarfärbemittel, am meisten bevorzugt ein Shampoo, eine Spülung, vorzugsweise eine Leave-on oder Leave-in Spülung, oder ein 2-in-1-Produkt ist.

4. Verwendung nach einem der vorhergehenden Ansprüche, wobei der Extrakt durch ein Verfahren erhalten wird oder erhältlich ist, das aus den Schritten der Extraktion von Zellmaterial von Isochrysis sp., vorzugsweise Tahiti-Isochrysis, mit einem flüssigen Extraktionsmittel besteht oder diese umfasst,
wobei die Extraktion umfasst
a) Aussetzen des Zellmaterials gegenüber dem Extraktionsmittel für bis zu 24 Stunden bei einer Temperatur von 50°C oder weniger, und
b) Entfernen des Zellmaterials, um einen Extrakt zu erhalten, wobei der Extrakt die Zusammensetzung ist oder zu der Zusammensetzung weiterverarbeitet wird,
und wobei vorzugsweise das Zellmaterial von Isochrysis sp. und/oder Tahiti Isochrysis durch ein Verfahren erhältlich ist oder erhalten wird, das aus den Schritten besteht oder diese umfasst:
i) Kultivierung von Zellen von Isochrysis sp. und/oder vorzugsweise von Tahiti Isochrysis,
ii) Ernten der Zellen, um vollständig oder weitgehend intaktes Zellmaterial zu erhalten,
iii) gegebenenfalls einmaliges oder mehrmaliges Waschen des Zellmaterials aus Schritt ii), um gewaschenes, im Wesentlichen oder vollständig intaktes Zellmaterial zu erhalten,
iv) gegebenenfalls Gefriertrocknen des Zellmaterials aus Schritt ii) und/oder Schritt iii), falls vorhanden.

5. Verwendung nach Anspruch 4, wobei das flüssige Extraktionsmittel ausgewählt ist aus der Gruppe bestehend aus Hexan, Ethylacetat, Wasser, Methanol und Mischungen von zwei oder mehreren dieser Extraktionsmittel.

6. Verwendung nach Anspruch 4 oder 5, wobei die Extraktion eine ein-, zwei-, drei- oder viermalige Wiederholung der Schritte a) und b) umfasst, und wobei in jedem Schritt a) das im jeweils vorhergehenden Schritt b) entfernte Zellmaterial verwendet wird und die Extrakte der Schritte b) kombiniert werden.

7. Verwendung nach einem der Ansprüche 4 bis 6, wobei für die Extraktion Zellmaterial in Schritt b) einer vorherigen Extraktion mit einem anderen Extraktionsmittel erhalten wird.

8. Verwendung nach einem der Ansprüche 4 bis 7, wobei das im ersten Schritt a) verwendete Zellmaterial gefriergetrocknete Isochrysis sp. ist, vorzugsweise Tahiti Isochrysis.

9. Verwendung nach einem der vorhergehenden Ansprüche, wobei die Zusammensetzung 0,0005 bis 1 Gew.-% und am meisten bevorzugt 0,001 bis 0,01 Gew.-% Extrakt, vorzugsweise Trockenextrakt, bezogen auf das Gesamtgewicht der Zusammensetzung umfasst.

10. Verwendung nach einem der vorhergehenden Ansprüche, wobei die Zusammensetzung auch 1,2-Pentandiol enthält, vorzugsweise in einer Menge im Bereich von 0,001 bis 99,9 Gew.-%, bevorzugter 10 bis 99,9 Gew.-%, noch bevorzugter 25 bis 99,8 Gew.-% und am meisten bevorzugt 50 bis 99,7 Gew.-%, bezogen auf das Gesamtgewicht der Zusammensetzung.

11. Verfahren zur Beeinflussung oder Modifizierung der Haarfaser von menschlichem Haar, bestehend aus oder umfassend die folgenden Schritte:
a) Bereitstellung einer Zusammensetzung, die aus einem Extrakt von Isochrysis sp., vorzugsweise von Tahiti Isochrysis, besteht oder diesen enthält,
wobei es sich bei der Zusammensetzung vorzugsweise um ein Haarpflegeprodukt, bevorzugter um ein Shampoo, eine Spülung, vorzugsweise ein Leave-on oder Leave-in-Spülung, ein 2-in-1-Produkt, ein Spitzenreparaturprodukt, eine Haarkur, ein Haartonikum, eine Haarlotion, eine Haarspülung, eine Stylingcreme, eine Haarfestigerzusammensetzung, vorzugsweise in Form eines Gels, Schaums oder Sprays, eine Stylinghilfe, eine Blondierungszusammensetzung oder eine Haarfärbezusammensetzung, am meisten bevorzugt um ein Shampoo, eine Spülung, vorzugsweise eine Leave-on oder Leave-in-Spülung, oder ein 2-in-1-Produkt handelt, und/oder
vorzugsweise, wobei die Zusammensetzung 0,00001 bis 10 Gew.-%, vorzugsweise 0,0005 bis 1 Gew.-% und am meisten bevorzugt 0,001 bis 0,01 Gew.-% Extrakt, vorzugsweise Trockenextrakt, bezogen auf das Gesamtgewicht der Zusammensetzung, umfasst, und/oder vorzugsweise, wobei die Zusammensetzung auch 1,2-Pentandiol umfasst, vorzugsweise in einer Menge im Bereich von 0,001 bis 99,9 Gew.-%.%, bevorzugter 10 bis 99,9 Gew.-%, bevorzugter 25 bis 99,8 Gew.-% und am meisten bevorzugt 50 bis 99,7 Gew.-%, bezogen auf das Gesamtgewicht der Zusammensetzung,
b) Auftragen der Zusammensetzung aus Schritt a) auf das menschliche Haar,
c) gegebenenfalls Spülen des menschlichen Haares, um Teile oder die gesamte Zusammensetzung aus dem menschlichen Haar zu entfernen,
d) gegebenenfalls Wiederholung der Schritte a) bis c), falls vorhanden, 1, 2, 3, 4, 5 oder mehr Mal,
e) gegebenenfalls Trocknen des menschlichen Haares,
wobei die Beeinflussung oder Modifizierung der Haarfaser des menschlichen Haares darstellt:
(i) Verbesserung der Kämmbarkeit von menschlichem Haar und/oder
(ii) Kontrolle des Volumens der menschlichen Haarfaser und/oder
(iii) Vermeidung oder Verringerung des Kräuselns von menschlichem Haar und/oder
(iv) Verbesserung der Zugfestigkeit der menschlichen Haarfaser und/oder
(v) Verringerung des Faserbruchs von menschlichem Haar, vorzugsweise während oder nach der Exposition der Haarfasern gegenüber physikalischen und/oder chemischen und/oder umweltbedingten Belastungen.

12. Verfahren nach Anspruch 11, Alternative (v), wobei die physikalische Belastung durch Kämmen, Trocknen, Erhitzen, Lockenwickeln oder Glätten der Haarfasern oder durch die Verwendung von Haltemitteln wie Haarspangen, Lockenwicklern oder Haargummis entsteht oder entstanden ist und/oder die chemische Belastung durch Haarpflegemittel wie Shampoos, Spülungen, Haarkuren, Haarwasser, Haarlotionen, Haarspülungen, Stylingcremes, Haarfestiger, Stylinghilfe, Blondiermittel oder Haarfärbemittel und/oder die Umweltbelastung durch Sonneneinstrahlung, industrielle Verschmutzung, Meerwasser oder chlorhaltiges Wasser entsteht oder entstanden ist.

13. Verfahren nach einem der Ansprüche 11 bis 12, wobei der Extrakt in Schritt a) durch ein Verfahren gemäß einem der Ansprüche 4 bis 8 erhalten wird oder erhältlich ist.

## Revendications

1. Utilisation d'une composition consistant en ou comprenant un extrait d'Isochrysis sp., de préférence d'Isochrysis de Tahiti, pour influencer ou modifier la fibre capillaire des cheveux humains, dans laquelle l'utilisation n'est pas une utilisation pour influencer ou modifier la croissance des cheveux humains ou la pigmentation des cheveux humains, dans laquelle la composition comprend entre 0,00001 et 10 % en poids d'extrait, de préférence d'extrait sec, par rapport au poids total de la composition, et
dans laquelle la composition est utilisée pour
(i) améliorer l'aptitude au peignage des cheveux humains et/ou
(ii) contrôler le volume des fibres capillaires humaines et/ou
(iii) éviter ou réduire les frisottis des cheveux humains et/ou
(iv) améliorer la résistance à la traction des fibres capillaires humaines et/ou
(v) diminuer la casse des fibres des cheveux humains, de préférence pendant ou après l'exposition de la fibre capillaire à des contraintes physiques et/ou chimiques et/ou environnementales.

2. Utilisation selon la revendication 1, variante (v), dans laquelle la contrainte physique provient ou avait pour origine le peignage, le séchage, le chauffage, le bouclage ou le lissage des fibres capillaires, ou l'utilisation de moyens de maintien tels que des pinces à cheveux, des bigoudis ou des élastiques à cheveux, et/ou le stress chimique provient ou avait pour origine des produits de soins capillaires tels que des shampooings, des après-shampoings, des cures de traitement capillaire, des toniques capillaires, des lotions capillaires, des rinçages capillaires, des crèmes coiffantes, des compositions fixatrices pour cheveux, des aides au coiffage, des compositions de décoloration ou des compositions de coloration capillaire, et/ou le stress environnemental provient ou avait pour origine l'exposition au soleil, à la pollution industrielle, à l'eau de mer ou à l'eau chlorée.

3. Utilisation selon l'une quelconque des revendications précédentes, dans laquelle la composition est un produit de soin capillaire, plus préférentiellement est un shampooing, un après-shampooing, de préférence un après-shampooing sans rinçage, un produit 2 en 1, un produit réparateur de pointes, une cure de traitement capillaire, un tonique capillaire, une lotion capillaire, un rinçage capillaire, une crème coiffante, une composition fixatrice pour cheveux, de préférence formulée en tant que gel, mousse ou spray, une aide au coiffage, une composition de décoloration ou une composition de coloration capillaire, le plus préférablement est un shampooing, un après-shampooing, de préférence un après-shampooing sans rinçage, ou un produit 2 en 1.

4. Utilisation selon l'une quelconque des revendications précédentes, dans laquelle l'extrait est obtenu ou peut être obtenu par un procédé consistant en ou comprenant les étapes d'extraction de matériau cellulaire d'Isochrysis sp., de préférence l'Isochrysis de Taihiti, avec un agent d'extraction liquide,
dans laquelle l'extraction comprend
a) exposition du matériau cellulaire à l'agent d'extraction pendant jusqu'à 24 h, à une température de 50 °C ou moins, et
b) élimination du matériau cellulaire pour obtenir un extrait, l'extrait étant la composition ou étant traité ultérieurement pour obtenir la composition,
et dans laquelle, de préférence, le matériau cellulaire d'Isochrysis sp. et/ou d'Isochrysis de Tahiti est susceptible d'être obtenue ou est obtenue par un procédé consistant en ou comprenant les étapes consistant à:
i) cultiver des cellules d'Isochrysis sp. et/ou de préférence des cellules d'Isochrysis de Tahiti,
ii) récolter les cellules pour obtenir un matériau cellulaire complètement ou pour l'essentiel intact,
iii) le cas échéant laver une ou plusieurs fois le matériau cellulaire de l'étape ii) pour obtenir du matériau cellulaire lavé, pour l'essentiel ou complètement intact,
iv) le cas échéant lyophiliser le matériau cellulaire de l'étape ii) et/ou de l'étape iii), si présente.

5. Utilisation selon la revendication 4, dans laquelle l'agent d'extraction liquide est choisi dans le groupe constitué par l'hexane, l'acétate d'éthyle, l'eau, le méthanol et des mélanges de deux ou plusieurs de ces agents d'extraction.

6. Utilisation selon la revendication 4 ou 5, dans laquelle l'extraction comprend la répétition des étapes a) et b) une, deux, trois ou quatre fois, et dans laquelle à chaque étape a) le matériau cellulaire éliminé à l'étape précédente respective b) est utilisé, et les extraits des étapes b) sont combinés.

7. Utilisation selon l'une quelconque des revendications 4 à 6, dans laquelle, pour l'extraction, du matériau cellulaire est obtenu à l'étape b) d'une extraction préalable avec un agent d'extraction différent.

8. Utilisation selon l'une quelconque des revendications 4 à 7, dans laquelle le matériau cellulaire utilisé dans la première étape a) est de l'Isochrysis sp. lyophilisé, de préférence de l'Isochrysis de Tahiti.

9. Utilisation selon l'une quelconque des revendications précédentes, dans laquelle la composition comprend entre 0,0005 et 1 % en poids et le plus préférablement entre 0,001 à 0,01 % en poids d'extrait, de préférence de l'extrait sec, par rapport au poids total de la composition.

10. Utilisation selon l'une quelconque des revendications précédentes, dans laquelle la composition comprend également du 1,2-pentanediol, de préférence en une quantité située dans la plage allant de 0,001 à 99,9 % en poids, plus préférablement de 10 à 99,9 % en poids, plus préférablement de 25 à 99,8 % en poids, et le plus préférablement de 50 à 99,7 % en poids, par rapport au poids total de la composition.

11. Procédé destiné à influencer ou à modifier la fibre capillaire des cheveux humains consistant en ou comprenant les étapes suivantes consistant à:
a) fournir une composition consistant en ou comprenant un extrait d'Isochrysis sp., de préférence d'Isochrysis de Tahiti,
de préférence dans lequel la composition est un produit de soin capillaire, plus préférentiellement est un shampooing, un après-shampooing, de préférence un après-shampooing sans rinçage, un produit 2 en 1, un produit réparateur de pointes, une cure de traitement capillaire, un tonique capillaire, une lotion capillaire, un rinçage capillaire, une crème coiffante, une composition fixatrice pour cheveux, de préférence formulée en tant que gel, mousse ou spray, une aide au coiffage, une composition de décoloration ou une composition de coloration capillaire, le plus préférablement est un shampooing, un après-shampooing, de préférence un après-shampooing sans rinçage, ou un produit 2 en 1 et/ou
de préférence dans lequel la composition comprend entre 0,00001 et 10 % en poids, de préférence entre 0,0005 et 1 % en poids et le plus préférablement entre 0,001 et 0,01 % en poids d'extrait, de préférence d'extrait sec, par rapport au poids total de la composition, et/ou de préférence dans lequel la composition comprend également du 1 ,2-pentanediol, de préférence en une quantité située dans la plage allant de 0,001 à 99,9 % en poids, plus préférablement de 10 à 99,9 % en poids, plus préférablement de 25 à 99,8 % en poids, et le plus préférablement de 50 à 99,7 % en poids, par rapport au poids total de la composition,
b) appliquer la composition de l'étape a) sur les cheveux humains,
c) le cas échéant rincer les cheveux humains pour éliminer des parties ou l'ensemble de la composition des cheveux humains,
d) le cas échéant répéter les étapes a) à c), si elles sont présentes, 1, 2, 3, 4, 5 fois ou plus,
e) le cas échéant sécher les cheveux humains,
dans lequel l'influence ou la modification de la fibre capillaire des cheveux humains est:
(i) une amélioration de l'aptitude au peignage des cheveux humains et/ou
(ii) un contrôle du volume des fibres capillaires humaines et/ou
(iii) une prévention ou une réduction des frisottis des cheveux humains et/ou
(iv) une amélioration de la résistance à la traction des fibres capillaires humaines et/ou
(v) une diminution de la casse des fibres des cheveux humains, de préférence pendant ou après l'exposition des fibres capillaires à des contraintes physiques et/ou chimiques et/ou environnementales.

12. Procédé selon la revendication 11, variante (v), dans laquelle la contrainte physique provient ou avait pour origine le peignage, le séchage, le chauffage, le bouclage ou le lissage des fibres capillaires, ou l'utilisation de moyens de maintien tels que des pinces à cheveux, des bigoudis ou des élastiques à cheveux, et/ou le stress chimique provient ou avait pour origine des produits de soins capillaires tels que des shampooings, des après-shampoings, des cures de traitement capillaire, des toniques capillaires, des lotions capillaires, des rinçages capillaires, des crèmes coiffantes, des compositions fixatrices pour cheveux, des aides au coiffage, des compositions de décoloration ou des compositions de coloration capillaire, et/ou le stress environnemental provient ou avait pour origine l'exposition au soleil, à la pollution industrielle, à l'eau de mer ou à l'eau chlorée.

13. Procédé selon l'une quelconque des revendications 11 à 12, dans lequel l'extrait à l'étape a) est obtenu ou susceptible d'être obtenu par un procédé tel que défini dans l'une quelconque des revendications 4 à 8.
